(19) Europäisches Patentamt
European Patent Office
Office européen des brevets

(11) Veröffentlichungsnummer: **0 160 842 B1**

# (12) EUROPÄISCHE PATENTSCHRIFT

(45) Veröffentlichungstag der Patentschrift: **22.06.94**

(51) Int. Cl.[5]: **C07D 457/12**, C07D 457/02, A61K 31/475

(21) Anmeldenummer: **85104073.3**

(22) Anmeldetag: **03.04.85**

Die Akte enthält technische Angaben, die nach dem Eingang der Anmeldung eingereicht wurden und die nicht in dieser Patentschrift enthalten sind.

## (54) 2-substituierte Ergolin-Derivate, Verfahren zu ihrer Herstellung und ihre Verwendung als Arzneimittel.

(30) Priorität: **09.04.84 DE 3413657**
**09.04.84 DE 3413658**
**09.04.84 DE 3413659**
**09.04.84 DE 3413660**

(43) Veröffentlichungstag der Anmeldung:
**13.11.85 Patentblatt 85/46**

(45) Bekanntmachung des Hinweises auf die Patenterteilung:
**22.06.94 Patentblatt 94/25**

(84) Benannte Vertragsstaaten:
**AT BE CH DE FR GB IT LI LU NL SE**

(56) Entgegenhaltungen:
**DE-A- 2 365 974**
**DE-A- 2 407 904**
**FR-A- 2 516 083**

**CHEMICAL ABSTRACTS Band 101, Nr. 13, 24. September 1984, Seite 686, Zusammenfassung Nr. 111243e, Columbus, Ohio, US; J. BENES et al.: "2,8-disubstituted 6-methylergoline-I derivatives"**

(73) Patentinhaber: **SCHERING AKTIENGESELLSCHAFT**

**D-13342 Berlin(DE)**

(72) Erfinder: **Sauer, Gerhard, Dr.**
**Königsbacher Zeile 47A**
**D-1000 Berlin 28(DE)**
Erfinder: **Huth, Andreas, Dr.**
**Kyllmannstrasse 15**
**D-1000 Berlin 39(DE)**
Erfinder: **Wachtel, Helmut, Dr.**
**Suarezstrasse 22**
**D-1000 Berlin 19(DE)**
Erfinder: **Schneider, Herbert Hans, Dr.**
**Duisburger Strasse 20**
**D-1000 Berlin 15(DE)**

**EP 0 160 842 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS Band 97, Nr. 15, 11. Oktober 1982, Seite 750, Zusammenfassung Nr. 127874w, Columbus, Ohio, US; J. BENES et al.: "Ergot alkaloids. LXIII. Wittig type reactions of 2-formylated derivatives of 6-methylergoline"

CHEMICAL ABSTRACTS Band 97, Nr. 7, 16. August 1982, Seite 679, Zusammenfassung Nr. 56099j, Columbus, Ohio, US; J. BENES et al.: "Ergot alkaloids. LXII. 2-formyl derivatives of 6-methylergoline-I"

## Beschreibung

Die Erfindung betrifft neue Ergolinderivate der Formel I, die in 2-Stellung substituiert sind, deren Herstellung nach an sich bekannten Methoden und ihre Verwendung als Arzneimittel auf Basis dieser Verbindungen.

In 2-Stellung mit einem 1,3-Dithiolan-2-yl-, Formyl- oder 2-Ethoxycarbonylvinyl-Rest substituierte 6,8β-Dimethylergoline sind aus Chemical Abstracts (1982), 97: 56099 j und aus Chemical Abstracts (1982), 97: 127874 w bekannt. Die erfindungsgemäßen Verbindungen haben die allgemeine Formel I

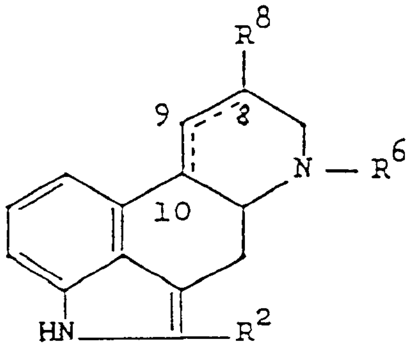

(I),

worin

$C_8$---$C_9$ und $C_9$---$C_{10}$ eine CC-Einfach- oder eine C=C-Doppelbindung, jedoch keine kumulierte Doppelbindung bedeutet und der Substituent in 8-Stellung α- oder β-ständig ist, wenn $C_8$---$C_9$ eine CC-Einfachbindung bedeutet,

$R^2$ CN,SR, SOR,

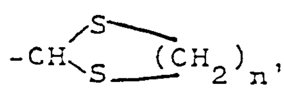

wobei n = 2 oder 3 ist,

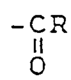

und -C(HOH)R mit R in der Bedeutung von
H oder $C_{1-4}$-Alkyl,
die Gruppierung COR' und CSR'
(R' = OH, $OC_{1-4}$-Alkyl, Benzyloxy, $NH_2$ oder NHR''),
die Gruppierung $CH_2$-$CH_2$-$CO_2R''$
(R'' = $C_{1-4}$-Alkyl),
die Gruppierung C≡C-R''' und HC=CH-R'''
(R''' = Wasserstoff, $C_{1-4}$-Alkyl, Phenyl, $CH_2OH$, $CR''_2OH$,

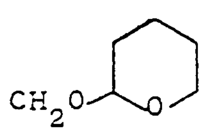

,

$CO_2R''$, $CH_2NR''_2$, $SiMe_2R''$) und
$C_{1-3}$-Alkyl, das mit OH oder Phenyl substituiert sein kann,

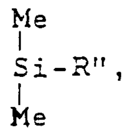

wobei R'' $C_{1-4}$-Alkyl
bedeutet,
$R^6$ $C_{1-4}$-Alkyl und
$R^8$ Methyl oder die Gruppierung $NH\text{-}CO\text{-}NEt_2$ oder $NH\text{-}CS\text{-}NEt_2$ darstellen, und deren Salze, wobei $C_8\underline{---}C_9$ eine Doppelbindung darstellt, falls $R^8$ $CH_3$ ist.

Niedere Alkylreste mit bis zu 3 C-Atomen sind solche, die sich von aliphatischen Kohlenwasserstoffen ableiten, wie z.B. Methyl, Ethyl, n-Propyl und Isopropyl.

Alkylreste mit bis zu 4 C-Atomen sind darüberhinaus z.B. n-Butyl, Isobutyl und tert.-Butyl.

Die Salze der erfindungsgemäßen Verbindungen der Formel I sind Säureadditionssalze und leiten sich von physiologisch unbedenklichen Säuren ab. Solche physiologisch unbedenklichen Säuren sind anorganische Säuren, wie beispielsweise Chlorwasserstoffsäure, Salpetersäure, Phosphorsäure, Schwefelsäure, Bromwasserstoffsäure, Jodwasserstoffsäure, salpetrige Säure oder phosphorige Säure, oder organische Säuren, wie beispielsweise aliphatische Mono- oder Dicarbonsäuren, phenylsubstituierte Alkancarbonsäuren, Hydroxyalkancarbonsäuren oder Alkandicarbonsäuren, aromatische Säuren oder aliphatische oder aromatische Sulfonsäuren. Physiologisch unbedenkliche Salze dieser Säuren sind daher z.B. das Sulfat, Pyrosulfat, Bisulfat, Sulfit, Bisulfit, Nitrat, Phosphat, Monohydrogenphosphat, Dihydrogenphosphat, Metaphosphat, Pyrophosphat, Chlorid, Bromid, Jodid, Fluorid, Acetat, Propionat, Decanoat, Caprylat, Acrylat, Formiat, Isobutyrat, Caproat, Heptanoat, Propiolat, Malonat, Succinat, Suberat, Sebacat, Fumarat, Maleat, Mandelat, Butin-1.4-dioat, Hexin-1.6-dioat, Benzoat, Chlorbenzoat, Methylbenzoat, Dinitrobenzoat, Hydroxybenzoat, Methoxybenzoat, Phthalat, Terephthalat, Benzolsulfonat, Toluolsulfonat, Chlorbenzolsulfonat, Xylolsulfonat, Phenylacetat, Phenylpropionat, Phenylbutyrat, Citrat, Lactat, $\beta$-Hydroxybutyrat, Glycollat, Malat, Tartrat, Methansulfonat, Propansulfonat, Naphthalin-1-sulfonat oder Naphthalin-2-sulfonat.

Im Vergleich zu bekannten, in 2-Stellung unsubstituierten Ergolinen, wie z.B. dem Lisurid oder dem Tergurid, zeichnen sich die erfindungsgemäßen Verbindungen der Formel I durch eine zentral dopaminerge und/oder $\alpha_2$-Rezeptor-blockierende Wirksamkeit aus.

Die zentrale $\alpha_2$-Rezeptorblockade, z.B. des 1.1-Diethyl-3-(6-methyl-2-methylthio-8$\alpha$-ergolinyl)-harnstoffs (A) und des 1.1-Diethyl-(6-methyl-2-ethinyl-8$\alpha$-ergolinyl)-harnstoffs (B)
wurde in einem Interaktionstest mit dem $\alpha_2$-Rezeptoragonisten Clonidin an Mäusen nach einmaliger i.p.-Vorbehandlung dargestellt (Parameter: Aufhebung der durch Clonidin 0.1 mg/kg i.p. verursachten Hypothermie). Männliche NMRI-Mäuse wurden mit verschiedenen Dosen von A bzw. B die selbst nicht die Thermoregulation der Versuchstiere beeinflussen, bzw. mit Trägermedium vorbehandelt. 30 Minuten später erhielten alle Tiere Clonidin 0.1 mg/kg i.p. 60 Minuten nach Gabe von A oder B bzw. Trägermedium (= 30 Minuten nach Clonidin) wurde die Rektaltemperatur mit Hilfe einer Thermosonde gemessen. Während die mit Trägermedium vorbehandelten Mäuse eine Hypothermie aufwiesen, war an mit den erfindungsgemäßen Verbindungen A oder B vorbehandelten Tieren der körpertemperatursenkende Effekt des Clonidin dosisabhängig aufgehoben. Der clonidinantagonistische Effekt von A und der von B war bereits in der Dosierung 0.2 mg/kg statistisch signifikant.

Die zentrale Dopaminrezeptorblockade von A und B wurde in einem Interaktionstest mit dem Dopaminrezeptoragonisten Apomorphin an Mäusen nach einmaliger i.p.-Vorbehandlung dargestellt (Parameter: Aufhebung der durch Apomorphin 5 mg/kg i.p. verursachten Hypothermie). Männliche NMRI-Mäuse wurden mit verschiedenen Dosen von A und B,die selbst nicht die Thermoregulation der Versuchstiere beeinflussen, bzw. mit Trägermedium vorbehandelt. 30 Minuten später erhielten alle Tiere Apomorphin 5 mg/kg i.p. 60 Minuten nach Gabe von A oder B bzw. Trägermedium (= 30 Minuten nach Apomorphin) wurde die Rektaltemperatur mit Hilfe einer Thermosonde gemessen. Während die mit Trägermedium vorbehandelten Mäuse eine Hypothermie aufwiesen, war an mit A oder B vorbehandelten Tieren der körpertemperatursenkende Effekt des Apomorphin dosisabhängig aufgehoben. Der apomorphin-antagonistische Effekt von A und der von B war bereits in der Dosierung 0.2 mg/kg statistisch signifikant.

Aufgrund dieser Befunde können die erfindungsgemäßen Verbindungen daher als Neuroleptika zur Behandlung von Psychosen des schizophrenen Formenkreises oder als Antidepressiva verwendet werden.

Tabelle 1: Antagonistische Wirkung der Vorbehandlung (30 min i.p.) mit verschiedenen Dosen von 2-substituierten Ergolinharnstoffen auf die durch Clonidin (0.1 mg/kg i.p.) ausgelöste Hypothermie an Mäusen. Die Rektaltemperatur der Versuchstiere wurde 30 min nach Clonidin (= 60 min nach Prüfsubstanz) gemessen (x : $p < 0.05$, xx : $p < 0.01$ vs Kontrolle; Varianzanalyse/Dunnett-Test).

| Substanz | | Rektaltemperatur [°C] (Mittelwerte ± S.E.M.) Prüfsubstanzdosis [mg/kg] | | | | | | | | | |
|---|---|---|---|---|---|---|---|---|---|---|---|
| | n | Kontrolle | 0.05 | 0.1 | 0.2 | 0.39 | 0.78 | 1.56 | 3.13 | 6.26 | 12.5 |
| TDHL | 8 | 33.1 ± 0.2 | - | - | - | 33.6 ± 0.2 | 33.7 ± 0.3 | 34.1 ± 0.3** | 34.7 ± 0.2** | 34.8 ± 0.3** | - |
| 2-C≡CH-TDHL | 8 | 34.6 ± 0.2 | 33.9 ± 0.2 | 34.5 ± 0.2 | 35.3 ± 0.2 | 35.6 ± 0.3* | 35.7 ± 0.3** | 35.9 ± 0.2** | - | - | - |
| 2-$SCH_3$-TDHL | 8 | 33.6 ± 0.1 | 34.2 ± 0.1 | 34.2 ± 0.2 | 35.4 ± 0.3** | 35.3 ± 0.1** | 35.4 ± 0.2** | 35.4 ± 0.3** | 35.9 ± 0.3** | - | - |
| 2-$CH_3$-TDHL | 8 | 34.1 ± 0.2 | - | - | 34.3 ± 0.3 | 34.8 ± 0.2 | 35.2 ± 0.2** | 36.0 ± 0.2** | 35.9 ± 0.2** | - | - |
| 2-$SC_2H_5$-TDHL | 8 | 33.7 ± 0.2 | - | 34.4 ± 0.2 | 34.0 ± 0.3 | 34.9 ± 0.4* | 35.5 ± 0.6** | 34.7 ± 0.5 | 35.9 ± 0.2** | - | - |
| 2-$SCH(CH_3)_2$-TDHL | 8 | 34.0 ± 0.2 | - | 34.0 ± 0.2 | 34.8 ± 0.2 | 34.6 ± 0.2 | 35.3 ± 0.2** | 36.2 ± 0.3** | 37.0 ± 0.3** | - | - |
| 2-$SC_3H_7$-TDHL | 8 | 33.7 ± 0.1 | - | - | 34.1 ± 0.2 | 34.4 ± 0.2* | 34.6 ± 0.2** | 34.7 ± 0.2** | 35.8 ± 0.1** | - | - |

TDHL = 1.1-Diethyl-(6-methyl-8α-ergolinyl)-harnstoff

Tabelle 2: Antagonistische Wirkung der Vorbehandlung (30 min i.p.) mit verschiedenen Dosen von 2-substituierten Ergolinharnstoffen auf die durch Apomorphin (5 mg/kg i.p.) ausgelöste Hypothermie an Mäusen. Die Rektaltemperatur der Versuchstiere wurde 30 min nach Apomorphin (= 60 min nach Prüfsubstanz) gemessen (x : $p < 0.05$, xx : $p < 0.01$ vs Kontrolle; Varianzanalyse/Dunnett-Test).

| Substanz | | Rektaltemperatur [°C] (Mittelwerte ± S.E.M.) Prüfsubstanzdosis [mg/kg] | | | | | | | | | |
|---|---|---|---|---|---|---|---|---|---|---|---|
| | n | Kontrolle | 0.05 | 0.1 | 0.2 | 0.39 | 0.78 | 1.56 | 3.13 | 6.25 | 12.5 |
| TDHL | 8 | 32.5 ± 0.4 | - | - | - | - | 33.9 ± 0.5 | 33.8 ± 0.4 | 35.1 ± 0.5** | 35.5 ± 0.5** | 36.0 ± 0.4 |
| 2-C≡CH-TDHL | 8 | 34.0 ± 0.3 | 33.6 ± 0.5 | 33.7 ± 0.5 | 36.1 ± 0.4** | 36.6 ± 0.4** | 37.3 ± 0.2** | 36.7 ± 0.2** | 35.7 ± 0.3** | - | - |
| 2-$SCH_3$-TDHL | 8 | 32.9 ± 0.4 | 34.3 ± 0.5* | 33.9 ± 0.3 | 34.7 ± 0.4** | 35.8 ± 0.3** | 35.5 ± 0.2** | 35.5 ± 0.3** | - | - | - |
| 2-$CH_3$-TDHL | 8 | 33.7 ± 0.5 | - | - | 33.0 ± 0.4 | 33.2 ± 0.3 | 34.3 ± 0.3 | 34.1 ± 0.3 | 35.0 ± 0.2** | 35.5 ± 0.2** | - |
| 2-$SC_2H_5$-TDHL | 8 | 32.7 ± 0.3 | - | 33.3 ± 0.2 | 33.7 ± 0.4 | 34.1 ± 0.7 | 35.2 ± 0.5** | 34.8 ± 0.4** | 35.5 ± 0.4** | - | - |
| 2-$SCH(CH_3)_2$-TDHL | 8 | 33.1 ± 0.3 | - | - | 33.0 ± 0.3 | 33.0 ± 0.3 | 33.4 ± 0.5 | 34.3 ± 0.4 | 34.0 ± 0.4 | 34.2 ± 0.5 | - |
| 2-$SC_3H_7$-TDHL | 8 | 33.0 ± 0.6 | - | 32.9 ± 0.3 | 33.1 ± 0.3 | 33.5 ± 0.6 | 33.9 ± 0.5 | 33.9 ± 0.5 | 34.7 ± 0.6 | - | - |

TDHL = 1.1-Diethyl-(6-methyl-8α-ergolinyl)-harnstoff

Die Herstellung der Verbindungen der Formel I erfolgt nach an sich bekannten Methoden. Beispielsweise ist das Verfahren zur Herstellung von Verbindungen der Formel IA und deren Salze

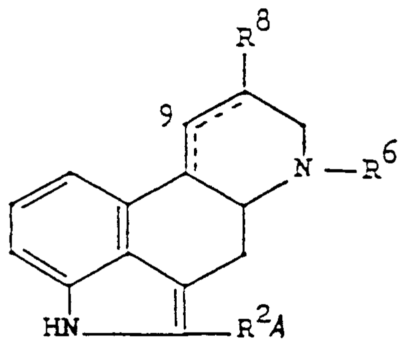

IA

worin

$C_8...C_9$ und $C_9...C_{10}$ eine Einfach- oder Doppelbindung, jedoch keine kumulierte Doppelbindung bedeuten und der Substituent in 8-Stellung α- oder β-ständig ist, wenn $C_8...C_9$ eine Einfachbindung bedeutet und $C_8...C_9$ eine Doppelbindung ist, wenn $R^8$ $CH_3$ bedeutet,

$R^6$ $C_{1-4}$-Alkyl,

$R^8$ Methyl, NH-CO-$NEt_2$ oder NH-CS-$NEt_2$ und

$R^{2A}$ CN, SR, SOR, -CO-R oder -CHOH-R mit R in der Bedeutung von H oder $C_{1-4}$-Alkyl ist,

dadurch gekennzeichnet, daß man eine Verbindung der Formel II

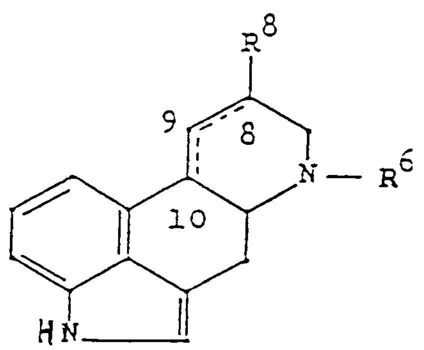

(II),

worin

$C_8...C_9$, $C_9...C_{10}$, $R^6$ und $R^8$ die oben angegebene Bedeutung haben,

mit einem elektrophilen Reagenz aus der Gruppe Chlorsulfonylisocyanat, Alkylthio-sulfoniumfluorborat oder Acylchlorid in Gegenwart einer Lewis-Säure umsetzt, gewünschtenfalls anschließend eine Carbonylgruppe am 2-Substituenten mit einem komplexen Metallhydrid reduziert oder eine Alkylthiogruppe mit Periodat oxidiert oder die Säureadditionssalze bildet.

Bedeutet der Substituent $R^2$ die Nitrilgruppe, so wird das Verfahren so durchgeführt, daß man zum Ergolin der Formel II in einem inerten Lösungsmittel wie Acetonitril oder Dimethylformamid in Gegenwart einer Base wie Trimethyl- oder Triethylamin das Chlorsulfonylisocyanat hinzu gibt (vgl. DE OS 2 365 974).

Die Anwendung einer inerten Gasatmosphäre ist zweckmäßig. Die Reaktion ist nach 10 - 50 Stunden bei Raumtemperatur abgeschlossen.

Bedeutet der Substituent $R^2$ die S-Alkyl- bzw. S-Methyl-Gruppe, so wird das Verfahren so durchgeführt, daß man das Ergolin der Formel II in einem inerten Lösungsmittel wie Dioxan oder Tetrahydrofuran das Sulfonium-Salz,wie z.B. das Dimethyl-methylthio-sulfoniumfluorborat, hinzu gibt. Die Reaktion ist nach 0,5 - 2 Stunden bei Raumtemperatur abgeschlossen.

Bedeutet der Substituent $R^2$ eine Acylgruppe, so wird das Verfahren so durchgeführt, daß das Ergolin der Formel II mit dem Acylchlorid in Gegenwart einer Lewis-Säure, wie z.B. Aluminiumtrichlorid, zur Reaktion gebracht wird. Ist das Acylchlorid jedoch nicht flüssig, kann als Solvens z.B. Nitrobenzol oder Chlorbenzol verwendet werden.

Die Reaktion wird vorzugsweise bei Temperaturen unter Raumtemperatur, wie -10 bis -5 °C, durchgeführt und ist nach 1-5 Stunden beendet.

Eine gegebenenfalls im Substituenten $R^2$ vorhandene Carbonylgruppe kann mit einem komplexen Metallhydrid wie z.B. Natriumborhydrid in einem protischen oder auch aprotischen Lösungsmittel, wie z.B.

Tetrahydrofuran, Acetonitril, Dioxan oder Dimethoxyethan, Methanol, Ethanol, Isopropanol, gegebenenfalls unter Zusatz von wasserfreiem Calciumchlorid, vorzugsweise bei der Siedetemperatur des verwendeten Lösungsmittels bzw. Reaktionsgemisches, zur Hydroxygruppe reduziert werden.

Die Oxidation der Methylthiogruppe zum Sulfoxid erfolgt mit einer wässrigen Lösung von Natriumperiodat in Acetonitril. Nach mehreren Stunden ist die Reaktion bei 50 °C beendet.

Beispielsweise ist das Verfahren zur Herstellung von Verbindungen der Formel IB und deren Salze

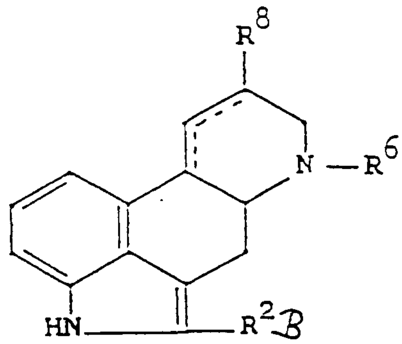

IB

worin

$C_8...C_9$ und $C_9...C_{10}$ eine Einfach- oder Doppelbindung, jedoch keine kumulierte Doppelbindung bedeuten und der Substituent in 8-Stellung $\alpha$- oder $\beta$-ständig ist, wenn $C_8...C_9$ eine Einfachbindung bedeutet und $C_8...C_9$ eine Doppelbindung ist, wenn $R^8$ $CH_3$ bedeutet,

$R^6$ $C_{1-4}$-Alkyl,

$R^8$ Methyl, NH-CO-$NEt_2$ oder NH-CS-$NEt_2$ und

$R^{2B}$ $C_{1-3}$-Alkyl, Hydroxyethyl, $C_{2-3}$-Alkenyl, -COOH -CONHR", -CSNHR", -COOR", -S-$C_{1-4}$-Alkyl oder -$SiMe_2C_{1-4}$-Alkyl bedeutet und R" $C_{1-4}$-Alkyl ist, dadurch gekennzeichnet, daß man eine Verbindung der Formel III

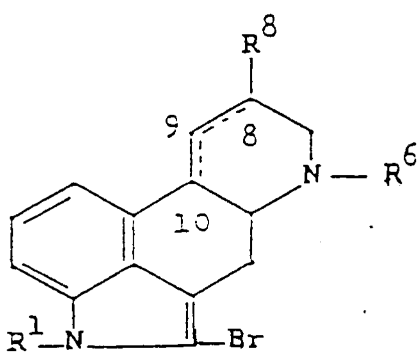

(III),

worin

$C_8...C_9$, $C_9...C_{10}$, $R^6$ und $R^8$ die oben angegebene Bedeutung haben und

$R^1$ eine Schutzgruppe darstellt, mit Lithiumalkyl oder mit Lithiumphenyl bei Temperaturen unter 0 °C umsetzt und das so erhaltene 2-Li-Ergolin-Derivat der allgemeinen Formel IV

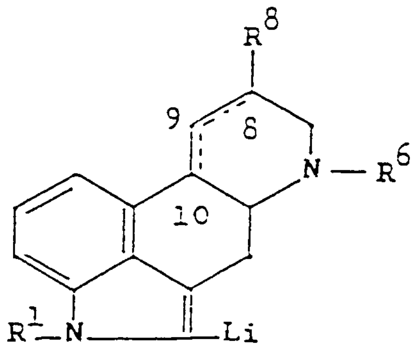

(IV),

worin

$C_8...C_9$, $C_9...C_{10}$, $R^1$, $R^6$ und $R^8$ die oben angegebene Bedeutung haben, mit einem elektrophilen Reagenz aus der Gruppe Kohlendioxid, Ethylenoxid, $C_{1-4}$-Alkylisocyanat, $C_{1-4}$-Alkylthioisocyanat, $C_{1-4}$-Alkyldimethylchlorsilan, $C_{1-3}$-Alkyl- und $C_{2-3}$-Alkenylbromid bzw. -jodid oder $C_{1-4}$Alkyldisulfide, bei tiefen Temperaturen von -110 bis -50 °C in einem inerten Lösungsmittel umsetzt und gewünschtenfalls eine freie 2-Carboxylfunktion mit einem aliphatischen Alkohol der Formel R"OH verestert oder die Schutzgruppe $R^1$ abspaltet oder die Säureadditionssalze bildet.

Hierzu wird in der ersten Stufe ein 2-Brom-ergolinderivat der Formel III mit einem Lithiumalkyl oder mit Lithiumphenyl bei tiefen Temperaturen in einem inerten Lösungsmittel zum entsprechenden 2-Lithium-ergolin-derivat der Formel IV umgesetzt. Als Lithiumalkyl kommt insbesondere tert.-Butyl-lithium in Frage.

Der in den Ausgangsverbindungen der Formel III in 1-Stellung auftretende Substituent $R^1$ hat die Funktion einer Schutzgruppe. Die Schutzgruppe $R^1$ kann eine Silylgruppe der Formel $Si(R')_2R$ darstellen, wobei R' einen niederen Alkylrest mit bis zu vier C-Atomen darstellt. $R^1$ kann aber auch eine Alkylgruppe mit bis zu sieben C-Atomen, eine Aralkylgruppe mit 7-9 C-Atomen, einen Acylrest mit 2-5 C-Atomen oder einen Arylsulfonylrest, wie den p-Toluolsulfonylrest, darstellen.

Alkylreste mit bis zu sieben C-Atomen sind neben den bereits genannten niederen Alkylresten z.B. n-Pentyl, n-Hexyl, 1-Methylpentyl und 2.2-Dimethyl-butyl.

Aralkylgruppen sind beispielsweise Benzyl und Phenethyl.

Acylgruppen mit 2-5 C-Atomen leiten sich von aliphatischen Carbonsäuren ab, wie Essigsäure, Propionsäure, Buttersäure, Capronsäure und Trimethylessigsäure.

Unter tiefen Temperaturen sind Temperaturen unter 0 °C, insbesondere solche im Bereich von -110 bis -70 °C, zu verstehen, wie sie z.B. durch Verwendung von festem Kohlendioxid oder von flüssigem Stickstoff in Methanol, Ether u.ä. Lösungsmitteln als Kühlmittel erreicht werden.

Als Lösungsmittel sind alle solche geeignet, die sich unter den genannten Reaktionsbedingungen inert verhalten. Genannt seien beispielsweise aliphatische und aromatische Kohlenwasserstoffe wie Hexan und Toluol oder Ether, wie Tetrahydrofuran, Dioxan und Diethylether. Bewährt hat sich der Zusatz einer stöchiometrischen Menge von Tetramethylethylendiamin bezogen auf das Lithiumalkyl.

Die Reaktion ist nach kurzer Zeit, d.h. nach etwa 2-10 Minuten, beendet.

Das so hergestellte 2-Lithium-ergolin-derivat der Formel IV ist bei tiefen Temperaturen stabil. Die Reaktionslösung wird nicht weiter aufgearbeitet und gleich in die nächste Reaktionsstufe eingesetzt. In dieser Reaktionsstufe wird das 2-Lithium-ergolin-derivat mit einem elektrophilen Reagenz, wie z.B. Kohlendioxid, Ethylenoxid, $C_{1-4}$-Alkylisocyanat, $C_{1-4}$-Alkylthioisocyanat, $C_{1-4}$-Alkyldimethylchlorsilan, $C_{1-3}$-Alkyl- und $C_{2-3}$-Alkenylbromid bzw. -jodid oder $C_{1-4}$Alkyldisulfide bei tiefen Temperaturen im Bereich von -110 bis -50 °C in einem inerten Lösungsmittel umgesetzt.

Stellt der Reaktant ein Gas dar, wird dieses eingeleitet oder fest, wie z.B. als festes Kohlendioxid und in einem Druckgefäß mit dem 2-Lithium-ergolin-derivat zur Reaktion gebracht.

Stellt der Substituent in 2-Stellung eine freie Carboxylfunktion dar, so kann diese gewünschtenfalls nach an sich bekannten Methoden mit einem aliphatischen Alkohol der Formel R"OH, worin R" einen $C_{1-4}$-Alkylrest darstellt, werestert werden.

Hierzu wird beispielsweise die Ergolin-2-carbonsäure mit dem Alkohol in Gegenwart einer anorganischen Säure wie Salzsäure oder Perchlorsäure bei Raumtemperatur zur Reaktion gebracht.

Beispielsweise ist das Verfahren zur Herstellung der Formel IC und deren Salze

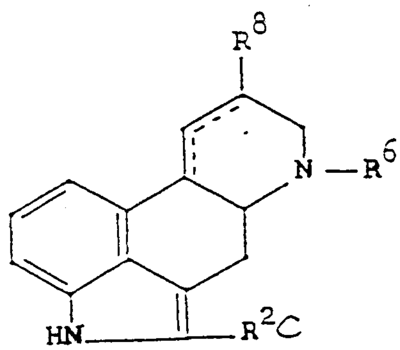
IC

worin
$C_8...C_9$ und $C_9...C_{10}$ eine Einfach- oder Doppelbindung, jedoch keine kumulierte Doppelbindung bedeuten und der Substituent in 8-Stellung α- oder β-ständig ist, wenn $C_8...C_9$ eine Einfachbindung bedeutet und $C_8...C_9$ eine Doppelbindung ist, wenn $R^8$ $CH_3$ bedeutet,
$R^6$ $C_{1-4}$-Alkyl,
$R^8$ Methyl, NH-CO-$NEt_2$ oder NH-CS-$NEt_2$ und
$R^{2C}$ die Gruppierung COOH, CO-O-Benzyl, CO-$NH_2$, oder $CH_2$-$CH_2$-$CO_2$R'' (R'' = $C_{1-4}$-Alkyl),
die Gruppierung C≡C-R''' oder HC = CH-R'''
(R''' = Wasserstoff, $C_{1-4}$-Alkyl, Phenyl, $CH_2OH$, $CR''_2OH$,

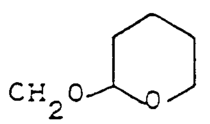
,

$CO_2R''$, $CH_2NR''_2$, $SiMe_2R''$)
$C_{2-3}$-Alkyl, mit Phenyl substituiertes Ethyl oder mit OH substituiertes Propyl bedeutet,
dadurch gekennzeichnet, daß man eine Verbindung der Formel V

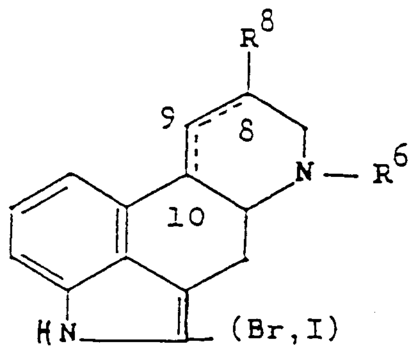
(V),

worin
$C_8$---$C_9$ , $C_9$---$C_{10}$, $R^6$ und $R^8$ die oben angegebene Bedeutung haben, mit einem elektrophilen Reagenz aus der Gruppe Benzylalkohol/Kohlenmonoxid, Acrylester $CH_2$ = CH-$CO_2$R'' oder Ethinylderivat CH≡C-R''', worin R'' und R''' die oben angegebene Bedeutung haben, in Gegenwart eines Palladium-Katalysators und eines sekundären oder tertiären Amins ohne Lösungsmittel oder in einem aprotischen, mit Wasser mischbaren Lösungsmittel bei Temperaturen oberhalb Raumtemperatur im Bereich von 40 °C bis zur Siedetemperatur des Reaktionsgemisches reagieren läßt, gewünschtenfalls anschließend die 2-Benzylgruppe mit Palladium unter Bildung der Carboxylverbindung hydriert oder mit Ammoniak zur 2-Carbonsäureamidgruppe umsetzt oder eine exocyclische Mehrfachbindung mit Raney-Nickel oder Palladium auf einem Träger zur CC-Einfachbindung oder Doppelbindung hydriert oder eine $SiMe_2$R''-Schutzgruppe mit einer

Base oder eine Tetrahydropyranyl-Schutzgruppe mit Pyridinium-p-toluolsulfonat abspaltet oder die Säureadditionssalze bildet.

Hierzu wird ein 2-Jod- oder 2-Brom-ergolin der Formel V mit einem elektrophilen Reagenz wie Benzylalkohol unter Kohlenmonoxidatmosphäre, einem Acrylester oder einem monosubstituierten Acetylen entweder ohne Lösungsmittel, in dem entsprechenden Amin oder in einem aprotischen, mit Wasser mischbaren Lösungsmittel, in Gegenwart eines sekundären oder tertiären Amins, bei Temperaturen oberhalb Raumtemperatur im Bereich von 40 °C bis zur Siedetemperatur des Reaktionsgemisches in Gegenwart eines Palladium-Katalysators, zur Reaktion gebracht.

Unter Acrylester sind solche Ester der Acrylsäure zu verstehen, die sich durch Veresterung mit einem niederen aliphatischen Alkohol mit bis zu 3 C-Atomen ergeben, wie z.B. der Acrylsäureethylester.

Mit monosubstituierten Acetylen werden solche Ethinylderivate bezeichnet, bei welchen ein H-Atom durch $C_{1-3}$-Alkyl, Phenyl, Hydroxymethyl, Tetrahydropyranyloxymethyl, $C_{1-3}$-Alkoxycarbonyl, Di-$C_{1-3}$-alkylaminomethyl und $C_{1-3}$-Alkyldimethylsilyl substituiert ist.

Als aprotische, mit Wasser mischbare Lösungsmittel seien beispielsweise genannt Dimethylformamid, N-Methylpyrrolidon, Tetrahydrofuran, Acetonitril und Dioxan.

Sekundäre und tertiäre Amine sind beispielsweise Dimethylamin, Diethylamin, Piperidin, Triethylamin und Tri-n-butylamin.

Als Palladium-Katalysatoren kommen Palladiumsalze und Palladium-Komplexverbindungen in Frage. Genannt seien z.B. Palladium-II-acetat, trans-Dichlor-bis-(tri-o-tolyl-phosphin)-palladium(II) oder trans-Dichlor-bis-(triphenylphosphin)-palladium(II) und Palladium(O)-tetrakis-triphenylphosphin. Der Katalysator wird in einer Menge von 0,01 bis 0,1 Mol bezogen auf das eingesetzte 2-Halogen-ergolin angewerdet.

Bewährt hat sich bei manchen der Reaktionen ein Zusatz von Kupfer-I-jodid oder von Tri-o-tolylphosphin.

Zweckmäßigerweise werden die Reaktionen unter Ausschluß der Luft, teilweise aber auch unter erhöhtem Druck, d.h. unter einer Inertgasatmosphäre und in einem Autoklaven, ausgeführt.

Ist eine -COOH-Gruppe erwünscht, so wird der entsprechende 2-Carbonsäurebenzylester in einem protischen Lösungsmittel, wie einem aliphatischen Alkohol, z.B. Methanol, in Gegenwart von feinverteiltem Palladium, wie Palladium-Mohr, mit Wasserstoff unter Normaldruck bei Raumtemperatur hydriert.

Der 2-Benzylester kann mit Ammoniak in einem protischen Lösungsmittel wie in einem Alkohol, vorzugsweise Ethylenglykol, bei erhöhter Temperatur zum entsprechenden Carbonsäureamid umgesetzt.

Enthält der Substituent in 2-Stellung eine exocyclische C=C-Doppelbindung oder C≡C-Dreifachbindung, so kann die ungesättigte Bindung z.B. mit Raney-Nickel oder Palladium/Kohle in einem aliphatischen Alkohol bei Raumtemperatur unter Normaldruck ganz oder teilweise leicht zum entsprechenden Hydrierungsprodukt reduziert werden.

Enthält der Ethinyl-Substituent in 2-Stellung eine Schutzgruppe, wie eine $SiMe_2R''$-Gruppe, so kann diese mit einer schwachen Base wie Natrium- oder Kaliumcarbonat bei Raumtemperatur, aber auch mit Säuren oder Fluoridionen (Cäsiumfluorid oder Tetrabutylammoniumfluorid) entfernt werden. Ist die Ethinylgruppe einseitig als Acetonanlagerungsprodukt geschützt, so wird die Schutzgruppe durch Kochen mit einer starken Base wie Kalium- oder Natriumhydroxid bei 100 °C entfernt.

Ist die Schutzgruppe der Tetrahydropyranylrest, so verwendet man zur Abspaltung eine Säure, wie z.B. Pyridinium-p-toluolsulfonat oder verdünnte Schwefelsäure, in einem Alkohol bei 70 - 100 °C.

Die so erhaltenen Verbindungen der Formel I werden entweder als freie Basen oder gewünschtenfalls in Form ihrer Säureadditionssalze, die durch Umsetzung mit einer physiologisch verträglichen Säure, wie z.B. Weinsäure oder Maleinsäure, erhalten werden, durch Umkristallisation und/oder Chromatographie gereinigt.

Zur Bildung von Salzen werden die Verbindungen der Formel I in wenig Methanol oder Methylenchlorid gelöst und mit einer konzentrierten Lösung der gewünschten Säure in Methanol bei Raumtemperatur versetzt.

Die für die Durchführung des erfinderischen Verfahrens benötigten Ausgangsverbindungen sind entweder bekannt oder können nach dem Fachmann bekannten Methoden hergestellt werden.

Herstellung des Ausgangsmaterials

1 mMol Tergurid wird in 20 ml wasserfreiem Dioxan gelöst, mit ca. 1,5 ml N-Jodsuccinimid bei Raumtemperatur versetzt und 30 Minuten lang gerührt. Anschließend gießt man das Reaktionsgemisch in eine gesättigte Bicarbonatlösung, extrahiert mit Methylenchlorid und trocknet die organische Phase mit Magnesiumsulfat. Nach Abdampfen des Lösungsmittels wird der Rückstand an Kieselgel chromatographiert. Man erhält in 76 %iger Ausbeute 1.1-Diethyl-3-(2-jod-6-methyl-8α-ergolinyl)-harnstoff
$[\alpha]_D$ = +37,3 ° c = 0,2 in Pyridin.

In analoger Weise erhält man aus Lisurid und N-Bromsuccinimid das 1.1-Diethyl-3-(2-brom-6-methyl-9.10-didehydro-8α-ergolinyl)-harnstoff (23 %)
$[\alpha]_D$ = +247 ° c = 0,2 in Pyridin.

In gleichfalls analoger Weise erhält man aus Lisurid und N-Jodsuccinimid das 1.1-Diethyl-3-(2-jod-6-methyl-9.10-didehydro-8α-ergolinyl)-harnstoff (20 %).

Aus 27 ml 15%igem n-Butyllithium in Hexan (67 mMol), 9,1 ml wasserfreiem Diisopropylamin und 40 ml wasserfreiem Tetrahydrofuran bereitet man sich unter Eiskühlung eine Lösung von Lithiumdiisopropylamid. Diese kühlt man auf -20 °C ab und tropft eine Lösung von 12,7 g Bromagroclavin (30 mMol) in 115 ml wasserfreiem Tetrahydrofuran hinzu, rührt 15 min bei dieser Temperatur und gibt dann 7,3 ml tert.-Butyldimethylsilylchlorid (48 mMol) in 15 ml wasserfreiem Tetrahydrofuran zu. Diese Mischung läßt man auf Raumtemperatur erwärmen und rührt 2 Tage lang. Man verteilt zwischen Essigester und Bicarbonatlösung, trocknet die organische Phase über Natriumsulfat und dampft ein. Der Rückstand wird an Kieselgel mit Methylenchlorid, dann mit Methylenclorid/Methanol chromatographiert. Man erhält 2-Brom-1-(tert-butyl-dimethyl-silyl)-8.9-didehydro-6.8-dimethyl-ergolin in 83 %iger Ausbeute.
$[\alpha]_D$ = -161 ° (0,5 % in Chloroform).

In analoger Weise werden die folgenden Verbindungen hergestellt:
3-(2-Brom-1-(tert.-butyl-dimethylsilyl)-9.10-didehydro-6-methyl-8α-ergolinyl)-1.1-diethylharnstoff.
Ausbeute: 72 % d. Th.

3-(2-Brom-1-(tert.-butyl-dimethylsilyl)-6-methyl-8α-ergolinyl)-1.1-diethylharnstoff.
Ausbeute: 63 % (aus Diisopropylether)
$[\alpha]_D$ = +6 ° (0,5 % aus Chloroform).

3-(2-Brom-1-(tert.-butyl-diphenylsilyl)-9.10-didehydro-6-methyl-8α-ergolinyl)-1.1-diethylharnstoff
Ausbeute: 29 %
$[\alpha]_D$ = +183 ° (0,5 % in Chloroform)
3-(2-Brom-1-(tert.-butyl-diphenylsilyl)-6-methyl-8α-ergolinyl)-1.1-diethylharnstoff
Ausbeute: 19 %
$[\alpha]_D$ = -0,6 ° (0,5 % in Chloroform)
3-(2-Brom-1-(tert.-butyl-dimethylsilyl)-9.10-didehydro-6-methyl-8α-ergolinyl)-1.1-diethyl-thioharnstoff
3-(2-Brom-1-(tert.-butyl-dimethylsilyl)-6-methyl-8α-ergolinyl)-1.1-diethyl-thioharnstoff
3-(2-Brom-1-(tert.-butyl-dimethylsilyl)-6-methyl-8β-ergolinyl)-1.1-diethylharnstoff, Ausbeute: 65 %
$[\alpha]_D$ = -80 ° (0,5 % in Chloroform)
3-(2-Brom-1-(tert.-butyl-dimethylsilyl)-6-n-propyl-8α-ergolinyl)-1.1-diethylharnstoff, Ausbeute: 77 %
$[\alpha]_D$ = -5 ° (0,5 % in Chloroform).

2,7 g Bromagroclavin (8,5 mMol) löst man in 400 ml Methylenchlorid, gibt 0,4 g Tetrabutylammoniumhydrogensulfat, 2,1 g pulverisiertes Kaliumhydroxid und 2,1 ml Trimethylessigsäurechlorid zu und rührt 30 Minuten bei Raumtemperatur. Dann gibt man Eis zu, rührt wieder 30 Minuten bei Raumtemperatur und schüttelt mit Bicarbonatlösung und weiterem Methylenchlorid aus. Nach dem Eindampfen und Chromatographieren an Kieselgel mit Methylenchlorid und Methanol erhält man 2,6 g 2-Brom-8.9-didehydro-6.8-dimethyl-1-(trimethylacetyl)-ergolin (76 % d.Th.).
$[\alpha]_D$ = -104 ° (0,5 % in Chloroform).

In analoger Weise werden die folgenden Acylverbindungen hergestellt:
2-Brom-8.9-didehydro-6.8-dimethyl-1-(tert.-butoxy-carbonyl)-ergolin, Ausbeute: 69 %.
$[\alpha]_D$ = -121 ° (0,5 % in Cloroform).

2-Brom-8.9-didehydro-6.8-dimethyl-1-(4-methoxy-2.3.6-trimethyl-phenylsulfonyl)-ergolin, Ausbeute: 74 % (nach Chromatographie) bzw. 41 % (nach Umkristallisation aus Methanol).
$[\alpha]_D$ = -142 ° (0,5 % in Chloroform).

2-Brom-8.9-didehydro-6.8-dimethyl-1-(2.4.6-trimethoxyphenylsulfonyl)-ergolin, Ausbeute : 81 % (aus Methanol).

Die Erfindung betrifft des weiteren ein neues Verfahren zur Herstellung von 2-(Trialkyl-silyl)-ergolinderivaten der Formel ID gemäß Patentanspruch 7. Beispielsweise ist das Verfahren zur Herstellung von Verbindungen der Formel ID und deren Salze

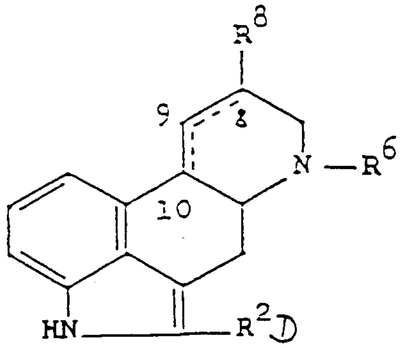

ID.

worin
$C_8...C_9$ und $C_9...C_{10}$ eine Einfach- oder Doppelbindung, jedoch keine kumulierte Doppelbindung bedeuten und der Substituent in 8-Stellung α- oder β-ständig ist, wenn $C_8...C_9$ eine Einfachbindung bedeutet und $C_8...C_9$ eine Doppelbindung ist, wenn $R^8$ $CH_3$ bedeutet,
$R^6$ $C_{1-4}$-Alkyl,
$R^8$ Methyl, NH-CO-$NEt_2$ oder NH-CS-$NEt_2$ und
$R^{2D}$ $SiMe_2$-$C_{1-4}$-Alkyl ist,
dadurch gekennzeichnet, daß man eine Verbindung der Formel VI

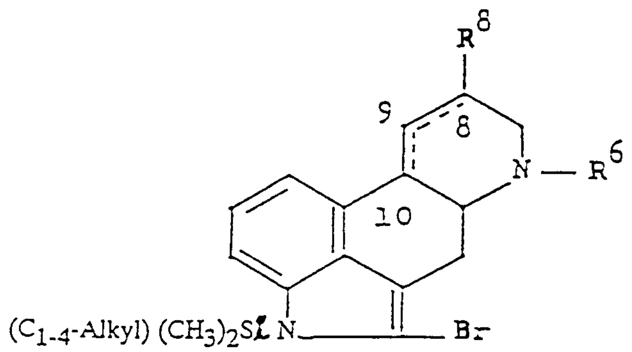

(VI),

worin
$C_8$---$C_9$, $C_9$---$C_{10}$, $R^6$ und $R^8$ die oben angegebene Bedeutung haben, mit einem Lithiumalkyl bei Temperaturen unter 0 °C gegebenenfalls in Gegenwert eines tert.-Amins umsetzt,
und gewünschtenfalls die Säureadditionssalze bildet.

2-(Trialkyl-silyl)-ergolinderivate können an sich durch elektrophile Substitution von in 2-Stellung metallierten Verbindungen hergestellt werden. Dieses Verfahren ist bei Ergolinderivaten bisher nicht beschrieben worden.

Es verläuft jedoch nur mit unbefriedigenden Ausbeuten.

Aufgabe der vorliegenden Erfindung war es, ein Verfahren zur Herstellung von 2-(Trialkyl-silyl)-ergolinderivaten bereitzustellen, welches bessere Ausbeuten liefert.

Es wurde nun überraschenderweise gefunden, daß die Herstellung von 2-(Trialkyl-silyl)-ergolinderivaten dann gut gelingt, wenn man 2-Brom-1-(trialkyl-silyl)-ergolinderivate in einem cyclischen Ether mit einem Lithiumalkyl umsetzt und zur entsprechenden 2-(Trialkyl-silyl)-ergolinverbindung umlagert.

Das erfindungsgemäße Verfahren wird so durchgeführt, daß man das 2-Brom-1-(trialkyl-silyl)-ergolin bei tiefen Temperaturen in einem cyclischen Ether als Lösungsmittel mit einem Lithiumalkyl umsetzt und anschließend zum entsprechenden 2-Silyl-ergolinderivat durch eine verlängerte Reaktionszeit umlagert.

Als Lithiumalkyl kommt insbesondere tert.-Butyl-Lithium in Frage. Es wird in einer Menge von 1 bis 5 Äquivalenten eingesetzt.

Unter tiefen Temperaturen sind Temperaturen unter 0 °C, insbesondere solche im Bereich vom -70 bis -20 °C zu verstehen, wie sie z.B. durch Kühlmittel wie festes Kohlendioxid in Methanol und/oder Methylenchlorid erreicht werden.

Als cyclische Ether sind insbesondere Tetrahydrofuran und Dioxan geeignet. Das Lösungsmittel wird im großen Überschuß eingesetzt, d.h. der etwa 10 - 500-fachen Menge.

In Abhängigkeit von der chemischen Struktur des als Ausgangsmaterial eingesetzten Ergolins kann ein tertiäres Amin wie Tetramethylethylendiamin dem Reaktionsgemisch zugesetzt werden.

Die Isomerisierung ist nach einigen Stunden, d.h. nach 2-8 Stunden, abgeschlossen.

Der Verlauf des erfindungsgemäßen Verfahrens war insofern überraschend, als die Umlagerungsreaktion in anderen bei Silylierungsreaktionen gebräuchlichen Lösungsmitteln wie Toluol oder Benzol nicht auftritt.

Das nachfolgende Beispiel soll diesen Tatbestand zeigen.

Man löste 800 mg 2-Brom-1-(tert.-butyl-dimethylsilyl)-8.9-didehydro-6.8-dimethyl-ergolin in 50 ml wasserfreiem Toluol, destillierte das Lösungsmittel im Vakuum ab und nahm mit 75 ml wasserfreiem, frisch destilliertem Toluol unter Argonatmosphäre auf. Zu dieser Lösung fügte man 1 ml wasserfreies Tetramethylethylendiamin hinzu und kühlte auf -90 °C ab. Dann wurde mit 6,0 ml 1,4 m tert.-Butyllithiumlösung in Hexan (8,4 mMol) versetzt und 5 Stunden gerührt. Dann gab man Wasser zu, schüttelte mit Methylenchlorid aus, trocknete die organische Phase mit Natriumsulfat und dampfte ein. Man isolierte in quantitativer Ausbeute 8.9-Didehydro-6.8-dimethyl-ergolin. Das gewünschte 8.9-Didehydro-6.8-dimethyl-2-trimethylsilyl-ergolin konnte durch NMR nicht nachgewiesen werden.

Zur Verwendung der erfindungsgemäßen Verbindungen als Arzneimittel werden diese in die Form eines pharmazeutischen Präparats gebracht, das neben dem Wirkstoff ein für die enterale oder parenterale Applikation geeignete pharmazeutische, organische oder anorganische inerte Trägermaterialien, wie z.B. Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, z.B. als Tabletten, Dragées, Suppositorien, Kapseln oder in flüssiger Form, z.B. als Lösungen, Suspensionen oder Emulsionen vorliegen. Gegebenenfalls enthalten sie darüberhinaus Hilfsstoffe wie Konservierungs-, Stabilisierungs-, Netzmittel oder Emulgatoren, Salze zur Veränderung des osmotischen Drucks oder Puffer.

Die nachfolgenden Beispiele sollen das erfindungsgemäße Verfahren erläutern.

## Beispiel 1

Man löst 680 mg Tergurid (2 mMol) in 120 ml Acetonitril und 5,5 ml Triethylamin und tropft unter Eiskühlung unter Argonatmosphäre eine Lösung von 3,5 ml Chlorsulfonylisocyanat in 40 ml Acetonitril zu. Man entfernt das Eisbad und läßt die Mischung zwei Tage bei Raumtemperatur stehen. Um die polaren Nebenprodukte besser isolieren zu können, wird mit 20 ml Diethylamin versetzt und drei Stunden bei Raumtemperatur gerührt. Dann wird mit Methylenchlorid verdünnt, mit 1n Natronlauge ausgeschüttelt und die Wasserphase nochmals extrahiert. Die vereinigten organischen Phasen werden mit Natriumsulfat getrocknet und eingedampft. Der Rückstand wird an Kieselgel chromatographiert mit einer Mischung von Methylenchlorid und Methanol. Man isoliert 229 mg (31 % d. Th.) rohen 3-(2-Cyan-6-methyl-8α-ergolinyl)-1.1-diethylharnstoff. Nach Kristallisation aus Essigester werden 92 mg als reine Substanz erhalten.
$[\alpha]_D$ = +72 ° (0,5 % in Pyridin).
Bei den polaren Nebenprodukten handelt es sich um Acylierungsprodukte in 1- und/oder 2-Stellung.

## Beispiel 2

Man löst 507 mg Lisurid (1,5 mMol) in 30 ml Tetrahydrofuran und gibt bei Raumtemperatur unter Stickstoff 588 mg Dimethyl-methylthio-sulfoniumfluorborat ( 3 mMol) zu. Nach 30 min Rühren bei Raumtemperatur verteilt man zwischen Methylenchlorid und Bicarbonatlösung, vereinigt die organischen Phasen, trocknet sie mit Natriumsulfat und dampft ein. Das Rohprodukt wird mit Methylenchlorid und Methanol an Kieselgel chromatographiert, die Ausbeute beträgt 506 mg (82 % d. Th.) 3-(9.10-Didehydro-6-methyl-2-methylthio-8α-ergolinyl)-1.1-diethylharnstoff, aus dem das weinsaure Salz hergestellt wird.
Ausbeute: 208 mg (55 % d.Th.).
$[\alpha]_D$ = +226 ° (0,5 % in Pyridin).

In analoger Weise werden hergestellt:
Aus Tergurid der
1.1-Diethyl-3-(6-methyl-2-methylthio-8α-ergolinyl)-harnstoff,
Ausbeute: 58 % (nach Chromatographie)
Weinsaures Salz (79 % Ausbeute)
$[\alpha]_D$ = +23 ° (0,5 % in Pyridin).

Aus 8.9-Didehydro-6.8-dimethyl-ergolin das 8.9-Didehydro-6.8-dimethyl-2-methylthio-ergolin.
Ausbeute: 95 % (nach Chromatographie)
Weinsaures Salz (40 % Ausbeute)

$[\alpha]_D$ = -172 ° (0,1 % in Pyridin).

Aus 3-(6-Methyl-8α-ergolinyl)-1.1-diethylthioharnstoff der 1.1-Diethyl-3-(6-methyl-2-methylthio-8α-ergolinyl)-thioharnstoff. Ausbeute: 26 %, $[\alpha]_D$ = +54 ° (0,5 % in Chloroform).

Aus 1.1-Diethyl-3-(6-methyl-8β-ergolinyl)-harnstoff den 1.1-Diethyl-3-(6-methyl-2-methylthio-8β-ergolinyl)-harnstoff, $[\alpha]_D$ = -100 ° (0,1 % in Chloroform). Ausbeute: 47 %.

Aus 1.1-Diethyl-3-(6-n-propyl-8α-ergolinyl)-harnstoff den 1.1-Diethyl-3-(2-methylthio-6-n-propyl-8α-ergolinyl)-harnstoff.
Ausbeute: 62 %, $[\alpha]_D$ = +7 ° (0,5 % in Chloroform).

Beispiel 3

3 ml (42 mMol) Acetylchlorid werden unter Eiskühlung mit 0,467 g (3,5 mMol) wasserfreiem Aluminiumchlorid und bei -10 °C mit 0,477 g (1,4 mMol) 3-(6-Methyl-8α-ergolinyl)-1.1-diethylharnstoff versetzt. Nach 2,5 Stunden Rühren bei -5 bis 0 °C setzt man 30 ml Hexan zu und dekantiert vom öligen Niederschlag ab, den man in Methylenchlorid/Wasser aufnimmt. Die organische Phase wird mit 10 %iger Natriumbicarbonatlösung mit gesättigter Natriumchloridlösung gewaschen, über Magnesiumsulfat getrocknet und eingeengt. Durch Säulenchromatographie über 150 g Kieselgel mit Methylenchlorid/Ethanol 10:1 und Umkristallisation aus Ethanol/Hexan erhält man 134 mg (25 %) 3-(2-Acetyl-6-methyl-8α-ergolinyl)-1.1-diethylharnstoff.
F. 114-116 °C
$[\alpha]_D$ = +51 ° (0,2 % in Pyridin).

In analoger Weise werden hergestellt:
2-Acetyl-8.9-didehydro-6.8-dimethylergolin
(F. 168-169 °C) Ethanol/Hexan
aus 8.9-Didehydro-6.8-dimethylergolin;
3-(2-Propionyl-6-methyl-8α-ergolinyl)-1.1-diethylharnstoff aus Tergurid
und
3-(2-Acetyl-9.10-didehydro-6-methyl-8α-ergolinyl)-1.1-diethylharnstoff
aus Lisurid.

Beispiel 4

57 mg (0,149 mMol) 3-(2-Acetyl-6-methyl-8α-ergolinyl)-1.1-diethylharnstoff in 4 ml absolutem Tetrahydrofuran werden mit 38 mg (1 mMol) Natriumborhydrid und 18 mg (0,162 mMol) wasserfreiem Calciumchlorid 2 Stunden am Rückfluß, erhitzt. Nach dem Abkühlen auf Raumtemperatur und Zugabe von Wasser rührt man 10 Minuten nach, destilliert das Tetrahydrofuran ab und versetzt mit Methylenchlorid. Durch Waschen der organischen Phase mit gesättigter Natriumchloridlösung und Trocknen über Magnesiumsulfat erhält man nach Einengen und Säulenchromatographie des Rohproduktes über 75 g Kieselgel mit Methylenchlorid/Ethanol = 5:1 26 mg (46,6 %) 3-[2-(1-Hydroxyethyl)-6-methyl-8α-ergolinyl]-1.1-diethylharnstoff als durchkristallisiertes Öl.
F.: ab 175 °C.

In analoger Weise werden hergestellt:
Aus 3-(2-Acetyl-9.10-didehydro-6-methyl-8α-ergolinyl) 1.1-diethylharnstoff den
3-[2-(1-Hydroxyethyl)-9.10-didehydro-6-methyl-8α-ergolinyl]-1.1-diethylharnstoff,
aus 3-(2-Propionyl-6-methyl-8α-ergolinyl)-diethylharnstoff den 3-[2-(1-Hydroxypropyl)-6-methyl-8α-ergolinyl]-1.1-diethylharnstoff
und aus 2-Acetyl-8.9-didehydro-6.8-dimethylergolin das 2-(1-Hydroxyethyl)-8.9-didehydro-6.8-dimethyl-ergolin.

Beispiel 5

Man löst 210 mg 1.1-Diethyl-3-(6-methyl-2-methylthio-8α-ergolinyl)-harnstoff in 20 ml Acetonitril und gibt portionsweise 0,5 g Natriummetaperiodat in 5 ml Wasser hinzu. Man rührt 16 Stunden bei 50 °C, verteilt den Rückstand zwischen Methylenchlorid und Wasser, trocknet die organische Phase mit Natriumsulfat und dampft ein. Der Rückstand wird an Kieselgel chromatographiert.
Ausbeute: 109 mg 1.1-Diethyl-3-(6-methyl-2-methylsulfinyl-8α-ergolinyl)-harnstoff.
$[\alpha]_D$ = +13 ° (0,5 % in Chloroform).

Beispiel 6

Man löst 800 mg 3-(2-Brom-1-(tert.-butyl-dimethylsilyl)-6-methyl-8α-ergolinyl)-1.1-diethylharnstoff (1,5 mMol) in 50 ml wasserfreiem Toluol, destilliert das Lösungsmittel im Vakuum ab und nimmt mit 75 ml wasserfreiem frisch destillierten Toluol unter Argonatmosphäre auf. Zu dieser Lösung fügt man 1 ml wasserfreies Tetramethylethylendiamin hinzu und kühlt auf -90 °C ab. Dann wird mit 6,0 ml 1,4 m tert.-Butyllithiumlösung in Hexan (8,4 mMol) versetzt und 2 min gerührt. Man erhält eine Lösung von 3-(1-tert.-Butyl-dimethylsilyl-2-lithium-6-methyl-8α-ergolinyl)-1.1-diethylharnstoff, die in die nächste Reaktionsstufe eingesetzt wird.

Die so hergestellte Lösung des 2-Lithiumergolins wird mit einer Lösung von 0,6 ml Methylisocyanat (9 mMol) in 4 ml Toluol versetzt und bei -70 °C 30 min lang gerührt. Dann gibt man Wasser zu, schüttelt mit Methylenchlorid aus, trocknet die organische Phase mit Natriumsulfat und dampft ein. Der Rückstand wird an Kieselgel mit Methylenchlorid und Methanol chromatographiert. Man isoliert 142 mg 8α-(3.3-Diethylureido)-6-methyl-ergolin-2-carbonsäuremethylamid (24 % d.Th.). Durch Kristallisation aus Methylenchlorid und Diisopropylether werden 87 mg (15 % d.Th.) erhalten.
$[\alpha]_D$ = +50 ° (0,2 % in Pyridin).

In analoger Weise werden die folgenden Verbindungen hergestellt:
9.10-Didehydro-8α-(3.3-diethylureido)-6-methyl-ergolin-2-carbonsäuremethylamid.
Ausbeute: 16 %
$[\alpha]_D$ = +284 ° (0,1 % in Pyridin).

8.9-Didehydro-6.8-dimethylergolin-2-carbonsäuremethylamid,
Ausbeute: 51 %
$[\alpha]_D$ = -162 ° (0,5 % in Chloroform).

Bei Ersatz des Methylisocyanats durch Methylisothiocyanat werden die folgenden Verbindungen erhalten:
8α-(3.3-Diethylureido)-6-methyl-ergolin-2-thiocarbonsäuremethylamid,
Ausbeute: 52 %
$[\alpha]_D$ = +22 ° (0,2 % in Pyridin)
9.10-Didehydro-8α-(3.3-diethylureido)-6-methyl-ergolin-2-thiocarbonsäuremethylamid,
Ausbeute: 22 %
$[\alpha]_D$ = +319 ° (0,1 % in Pyridin)
8.9-Didehydro-6.8-dimethyl-ergolin-2-thiocarbonsäuremethylamid,
Ausbeute: 58 %,
$[\alpha]_D$ = -328 ° (0,2 % in Pyridin).

Bei Ersatz des Methylisocyanats durch Trimethylsilyl-isocyanat:
8α-(3.3-Diethylureido)-6-methyl-ergolin-2-carbonsäureamid.
Ausbeute: 47 %; $[\alpha]_D$ = +42 ° (0,2 % in Pyridin).

Mit Trifluormethansulfonsäure-trimethylsilylester statt Methylisocyanat:
8.9-Didehydro-6.8-dimethyl-2-trimethylsilyl-ergolin,
Ausbeute: 15 %;
$[\alpha]_D$ = -177 ° (0,5 % in Chloroform) und
1.1-Diethyl-3-(6-methyl-2-trimethylsilyl-8α-ergolinyl)-harnstoff, Ausbeute: 35 %;
$[\alpha]_D$ = +30 ° (0,5 % in Chloroform).

Mit Methyliodid statt Methylisocyanat:
3-(2.6-Dimethyl-8α-ergolinyl)-1.1-diethylharnstoff.

Mit Kohlendioxid statt Methylisocyanat:
Eine Lösung von 2-Lithiumergolin, die wie oben beschrieben, zubereitet wird, wird rasch auf möglichst trockenes, festes $CO_2$ geschüttet. Die Mischung wird in einem Druckgefäß eingeschlossen, wo sie sich auf Raumtemperatur erwärmt. Am nächsten Morgen öffnet man vorsichtig und arbeitet den Inhalt wie üblich auf. Das Reaktionsprodukt wird chromatographisch gereinigt.

8.9-Didehydro-6.8-dimethyl-ergolin-2-carbonsäure,
Ausbeute: 60 %
$[\alpha]_D$ = -152 ° (0,1 % in Pyridin)
9.10-Didehydro-8α-(3.3-diethylureido)-6-methyl-ergolin-2-carbonsäure,
Ausbeute: 42 %
$[\alpha]_D$ = +187 ° (0,2 % in Methanol)
8α-(3.3-Diethylureido)-6-methyl-ergolin-2-carbonsäure,
Ausbeute: 48 %

$[\alpha]_D$ = +27 ° (0,2 % in Methanol).

Bei Ersatz des Methylisocyanats durch Dimethyldisulfid:
1.1-Diethyl-3-(6-methyl-2-methylthio-8α-ergolinyl)-harnstoff, Ausbeute: 67 %, davon das weinsaure Salz in in 80 % Ausbeute.
$[\alpha]_D$ = +23 ° (0,5 % in Pyridin).

Beispiel 7

Durch Veresterung der im Beispiel 6 erhaltenen freien Ergolin-2-carbonsäuren mit Methanol werden die betreffenden Methylester durch Stehenlassen bei Raumtemperatur in methanolischer Salzsäure erhalten:
8.9-Didehydro-6.8-dimethyl-ergolin-2-carbonsäuremethylester,
Ausbeute: 42 %
$[\alpha]_D$ = -266 ° (0,5 % in Chloroform);
9.10-Didehydro-8α-(3.3-diethylureido)-6-methyl-ergolin-2-carbonsäuremethylester,
Ausbeute: 46 %,
Weinsaures Salz, Ausbeute: 57 %,
$[\alpha]_D$ = +217 ° (0,1 % in Pyridin),
8α-(3.3-Diethylureido)-6-methyl-ergolin-2-carbonsäuremethylester,
Ausbeute: 50 %,
Weinsaures Salz, Ausbeute: 60 %,
$[\alpha]_D$ = +33 ° (0,1 % in Pyridin).

Beispiel 8

Man löst 730 mg 2-Brom-8.9-didehydro-6.8-dimethyl-1-(trimethylacetyl)-ergolin (1,5 mMol) unter Argonatmosphäre in 60 ml wasserfreiem Toluol, fügt zu dieser Lösung 1 ml wasserfreies Tetramethylethylendiamin hinzu und kühlt auf -90 °C ab. Man versetzt mit 1,4 ml tert.-Butyllithium-Lösung in Hexan (8,4 mMol) und rührt 2 Minuten. Die so erhaltene Lösung von 8.9-Didehydro-6.8-dimethyl-2-lithium-1-trimethylacetyl-ergolin wird rasch auf trockenes festes Kohlendioxid geschüttet und in ein Druckgefäß eingeschlossen. Am nächsten Morgen öffnet man vorsichtig und arbeitet wie üblich auf. Man erhält 8.9-Didehydro-6.8-dimethyl-ergolin-2-carbonsäure in 51%iger Ausbeute.
$[\alpha]_D$ = -151 ° (0,1 % in Pyridin).

Beispiel 9

Wie im Beispiel 6 beschrieben stellt man sich aus 3-(2-Brom-1-(tert.-butyl-dimethylsilyl)-6-methyl-8β-ergolinyl)-1.1-diethylharnstoff und tert. Butyllithium die entsprechende 2-Lithiumverbindung her. Diese wird statt mit Methylisocyanat mit Dimethyldisulfid umgesetzt zum 1.1-Diethyl-3-(6-methyl-2-methylthio-8β-ergolinyl)-harnstoff, Ausbeute: 54 %.
$[\alpha]_D$ = -100 ° (0,1 % in Methanol).

Beispiel 10

Analog Beispiel 6 erhält man aus 3-(2-Brom-1-(tert.-butyldimethylsilyl)-6-n-propyl-8α-ergolinyl)-1.1-diethylharn stoff mit tert.-Butyllithium die entsprechende Lithiumverbindung und aus dieser mit Dimethyldisulfid den 1.1-Diethyl-3-(2-methylthio-6-n-propyl-8α-ergolinyl)-harnstoff.
Ausbeute: 51 %; $[\alpha]_D$ = +6 ° (0,5 % in Chloroform).

Beispiel 11

300 mg (0,644 mMol) 3-(2-Jod-6-methyl-8α-ergolinyl)-1.1-diethylharnstoff werden in 4 ml Benzylalkohol nach Zusatz von 0,169 ml (0,709 mMol) Tri-n-butylamin erhitzt, unter Kohlenmonoxid-Atmosphäre mit 7 mg (0,031 mMol) Palladium-II-acetat versetzt und 2,5 Stunden unter kräftigem Rühren auf 100-110 °C gehalten. Nach dem Abkühlen auf Raumtemperatur wird die Reaktionslösung mit Essigester verdünnt und mit gesättigter Natriumhydrogencarbonatlösung und gesättigter Natriumchloridlösung ausgeschüttelt. Nach dem Trocknen über Magnesiumsulfat wird der Essigester unter vermindertem Druck und anschließend der Benzylalkohol im Hochvakuum abdestilliert. Aus dem Rohprodukt erhält man nach Säulenchromatographie an 150 g Kieselgel im System Dichlormethan/Ethanol = 10:1 und Umkristallisation aus Ethanol/Hexan 166

mg 8α-(3.3-Diethylureido)-6-methyl-ergolin-2-carbonsäure-benzylester (54,5 %).
F. 226-229 °C
$[\alpha]_D$ = +43,2 ° (c = 0,25 % in Pyridin).

Beispiel 12

104 mg (0,22 mMol) 8α-(3.3-Diethylureido)-6-methyl-ergolin-2 -carbonsäure-benzylester werden in 20 ml Methanol gelöst und nach Zusatz von 50 mg Palladium-Mohr 30 min bei Raumtemperatur unter Normaldruck hydriert. Nach Abfiltrieren des Katalysators und Einengen erhält man 8α-(3.3-Diethylureido)-6-methyl-ergolin-2-carbonsäure in 100 %iger Ausbeute.
F.: Zersetzung ab 230 °C.

Beispiel 13

150 mg (0,31 mMol) 8α-(3.3-Diethylureido)-6-methyl-ergolin-2-carbonsäurebenzylester werden in 3 ml einer gesättigten Lösung von Ammoniak in Ethylenglykol 3 Stunden auf 100 °C erwärmt. Nach Verdünnen mit Wasser wird mit Essigester extrahiert und die organische Phase mit gesättigter Kochsalzlösung gewaschen, über Magnesiumsulfat getrocknet, filtriert und eingeengt. Der Rückstand wird über Kieselgel mit Methylenchlorid/Ethanol = 8:1 chromatographiert.
Nach Verreiben in Ethanol/Hexan/Ether erhält man 90 mg 8α-(3.3-Diethylureido)-6-methylergolin)-2-carbonsäureamid vom Schmelzpunkt 171-173 °C.
$[\alpha]_D$ = +42,5 ° (c = 0,2 % in Pyridin).

Beispiel 14

261 mg (0,56 mMol) 3-(2-Jod-6-methyl-8α-ergolinyl)-1.1-diethylharnstoff, 7,0 mg (0,023 mMol) Tri-o-tolylphosphin, 4,7 mg (0,006 mMol) trans-Dichlor-bis-(tri-o-tolylphosphin)-palladium(II) und 0,075 ml (0,69 mMol) Acrylsäureethylester werden in 1,5 ml Dimethylformamid und 0,7 ml Triethylamin gelöst und nach Spülen mit Argon im Autoklaven 4 Stunden auf 100 °C erhitzt. Nach dem Einengen der Reaktionslösung unter vermindertem Druck wird in Essigester aufgenommen und mit gesättigter Kochsalzlösung gewaschen. Das nach Trocknen der Essigesterphase über Magnesiumsulfat und Einengen der Lösung erhaltene Rohprodukt wird über 150 g Kieselgel mit Dichlormethan/Ethanol 10:1 als Lösungsmittel chromatographiert. Man erhält 3-(2-Ethoxycarbonylvinyl-6-methyl-8α-ergolinyl)-1.1-diethylharnstoff.
Ausbeute: 115 mg (47 %)
F.: 223-225 °C (Ethanol/Hexan)
$[\alpha]_D$ = +115 ° (c = 0,2 % in Pyridin).

Beispiel 15

178 mg (0,406 mMol) 3-(2-Ethoxycarbonylvinyl)-6-methyl-8α-ergolinyl)-1.1-diethylharnstoff werden in 20 ml Ethanol gelöst und nach Zusatz von 0,1 g Raney-Nickel 1 Stunde bei Raumtemperatur unter Normaldruck hydriert. Nach dem Abfiltrieren des Katalysators erhält man durch Umkristallisation aus Essigester/Hexan 86 mg 3-[2-(2-Ethoxycarbonylethyl)-6-methyl-8α-ergolinyl]-1.1-diethylharnstoff (48 %).
F.: 160-161 °C.
$[\alpha]_D$ = +22 ° (c = 0,2 % in Pyridin).

Beispiel 16

933 mg (2,0 mMol) 3-(2-Jod-6-methyl-8α-ergolinyl)-1.1-diethylharnstoff werden in einem Gemisch von 5 ml Dimethylformamid und 10 ml Triethylamin gelöst und nach Zugabe von 0,610 ml (4,4 mMol) Ethinyltrimethylsilan, 19 mg (0,1 mMol) Kupfer(I)-jodid und 47,2 mg (0,06 mMol) trans-Dichlor-bis-(tri-o-tolylphosphin)-palladium(II) 3 Stunden unter Argon auf 60 °C erhitzt. Nach Einengen unter vermindertem Druck und Aufnehmen des Rückstandes in Essigester/Wasser wird die organische Phase mit gesättigter Natriumchloridlösung gewaschen, über Magnesiumsulfat getrocknet und eingedampft. Nach Säulenchromatographie über 150 g Kieselgel mit Methylenchlorid/Ethanol 10:1 als Elutionsmittel werden 486 mg 3-(2-Ethinyltrimethylsilyl-6-methyl-8α-ergolinyl)-1.1-diethylharnstoff (55,7 %) als Öl erhalten.

In analoger Weise werden hergestellt:
3-{6-Methyl-2-[3-(tetrahydropyran-2-yloxy)-propinyl]-8α-ergolinyl}-1.1-diethylharnstoff

F. 188 °C
$[\alpha]_D$ = +64 ° (c = 0,2 % in Pyridin);
3-(2-Propinyl-6-methyl-8α-ergolinyl)-1.1-diethylharnstoff,
3-[2-(1-Dimethylaminopropin-3-yl)-6-methyl-8α-ergolinyl]-1.1-diethylharnstoff,
1.1-Diethyl-3-[2-(3-hydroxy-3-methyl-1-butinyl)-6-methyl-8α-ergolinyl]-harnstoff, F.: ab 132-140 °,
$[\alpha]_D$ = + 67,8 ° (c = 0,2 % in Pyridin).
1.1-Diethyl-3-(2-phenylethinyl-6-methyl-8α-ergolinyl)-harnstoff, F.: 240-242 °, $[\alpha]_D$ = +107,5 ° (c = 0,2 % in Pyridin).
3-[2-(1-Carbethoxyethin-2-yl)-6-methyl-8α-ergolinyl]-1.1-diethylharnstoff,
3-(2-Trimethylsilylethinyl-6-methyl-8β-ergolinyl)-1.1-diethylharnstoff,
3-(2-Trimethylsilylethinyl-6-methyl-8α-ergolinyl)-1.1-diethylthioharnstoff,
8.9-Didehydro-6.8-dimethyl-2-phenylethinylergolin F.: ab 118 °C (unter Zersetzung)
und
3-(2-Trimethylsilylethinyl-6-n-propyl-8α-ergolinyl)-1.1-diethylharnstoff.

Beispiel 17

Ausgehend von 3-(2-Brom-6-methyl-8α-ergolinyl)-1.1-diethylharnstoff wird mit 3-(Tetrahydropyran-2-yloxy)-1-propin das 3-{9.10-Didehydro-6-methyl-2-[-3-(tetrahydropyran-2-yloxy)-1-propinyl]-8α-ergolinyl}-1.1-diethylharnstoff (25 %) und mit 3-Hydroxy-3-methyl-1-butin das 3-[9,10-Didehydro-2-(3-hydroxy-3-methyl-1-butinyl)-6-methyl-8α-ergolinyl]-1.1-diethylharnstoff (31 %) erhalten.

Beispiel 18

262 mg (0,6 mMol) 3-(2-Ethinyltrimethylsilyl-6-methyl-8α-ergolinyl)-1.1-diethylharnstoff werden in einem Gemisch aus 9 ml Ethanol und 1 ml Wasser gelöst und nach Zugabe von 104 mg (0,75 mMol) wasserfreiem Kaliumcarbonat 16 Stunden bei Raumtemperatur gerührt. Man destilliert das Lösungsmittel ab, nimmt in Essigester auf und schüttelt mit gesättigter Natriumchloridlösung aus. Nach dem Trocknen der Essigesterlösung über Magnesiumsulfat, Abdestillieren des Lösungsmittels und Säulenchromatographie des Rohproduktes an 150 g Kieselgel mit Toluol, Ethanol, Wasser im Verhältnis 80:20:1 als Elutionsmittel werden 81 mg 3-(2-Ethinyl-6-methyl-8α-ergolinyl)-1.1-diethylharnstoff (37 %) durch Umkristallisieren aus Ethanol/Hexan erhalten.
F.: 192 °C
$[\alpha]_D$ = 60,6 ° (c = 0,175 % in Pyridin)
In analoger Weise werden hergestellt:
3-(2-Ethinyl-6-methyl-8α-ergolinyl)-1.1-diethylthioharnstoff,
3-(2-Ethinyl-1.6-dimethyl-8α-ergolinyl)-1.1-diethylharnstoff
und
3-(2-Ethinyl-6-n-propyl-8α-ergolinyl)-1.1-diethylharnstoff, F.: 126 °C.
$[\alpha]_D$ = +65,0 ° (c = 0,13 % in Pyridin).

Beispiel 19

122 mg (0,29 mMol) 3-[9.10-Didehydro-2-(3-hydroxy-3-methyl-1-butinyl)-6-methyl-8α-ergolinyl]-1.1-diethylharnstoff werden in 15 ml absolutem Toluol nach Zugabe von 64 mg (1,6 mMol) gepulvertem Natriumhydroxid 2 Stunden unter Stickstoff am Rückfluß erhitzt. Anschließend wird das Lösungsmittel abdestilliert und der Rückstand in Essigester mit Wasser und gesättigter Kochsalzlösung ausgeschüttelt. Das aus der Essigesterlösung nach Trocknen mit Magnesiumsulfat und Einengen erhaltene Rohprodukt wird durch Säulenchromatographie an 80 g Kieselgel mit Essigester/Ethanol 2:1 als Elutionsmittel gereinigt. Durch Umfällen aus Essigester/Ether/Hexan erhält man 3-(9.10-Didehydro-2-ethinyl-6-methyl-8α-ergolinyl)-1.1-diethylharnstoff in 25 %iger Ausbeute.
$[\alpha]_D$ = +34 ° (c = 0,2 % in Pyridin).

Beispiel 20

213 mg (0,45 mMol) 3-{6-Methyl-2-[3-(tetrahydropyran-2-yl-oxy)-propinyl]-8α-ergolinyl}-1.1-diethylharnstoff werden in 10 ml Ethanol mit 2 ml Wasser und 176 mg (0,7 mMol) Pyridinium-p-toluolsulfonat 1 Stunde am Rückfluß unter Argon erhitzt. Nach Eindampfen und Verteilen in Essigester und gesättigter Bicarbonatlösung wird die organische Phase mit gesättigter Kochsalzlösung gewaschen, über Magnesiumsulfat getrocknet, filtriert und eingeengt. Der Rückstand wird über Kieselgel zunächst mit Essigester:Aceton = 1:1 und später mit Methylenchlorid: Aceton = 12:1 chromatographiert. Nach Umkristallisation aus Essigester/Hexan erhält man 90 mg (51 %) 3-[2-(1-Hydroxypropin-3-yl)-6-methyl-8α-ergolinyl]-1.1-diethylharnstoff.
F.: 151-154 °C
$[\alpha]_D$ = +71,2 ° (c = 0,21 % in Pyridin).

In analoger Weise wird hergestellt:
3-[2-(1-Hydroxypropin-3-yl)-9.10-didehydro-6-methyl-8α-ergolinyl]-1.1-diethylharnstoff.
$[\alpha]_D$ = +404 ° (c = 0,2 in Pyridin)
F.: >129 °C.

Beispiel 21

140 mg (0,084 mMol) 3-(2-Ethinyl-6-methyl-8α-ergolinyl)-1.1-diethylharnstoff werden in 20 ml Ethanol nach Zusatz von 0,1 g Raney-Nickel in 45 Minuten bei Raumtemperatur und Normaldruck hydriert. Nach Abfiltrieren des Katalysators und Einengen der Lösung wird aus Ethanol/Hexan umkristallisiert. Ausbeute: 119 mg 3-(2-Ethyl-6-methyl-8α-ergolinyl)-1.1-diethylharnstoff (84 %).
F.: 165-168 °C
$[\alpha]_D$ = +25 ° (c = 0,22 in Pyridin).

In analoger Weise werden hergestellt:
1.1-Diethyl-3-(6-methyl-2-phenethyl-8α-ergolinyl)-harnstoff, F.: 175-177 °, $[\alpha]_D$ = +26 ° (c = 0,1 % in Pyridin).

Beispiel 22

200 mg 3-(2-Ethinyl-6-methyl-8α-ergolinyl)-1.1-diethylharnstoff werden in 35 ml Ethanol mit 200 mg Palladium/Calciumcarbonat (2 %) und 200 mg Chinolin bei Raumtemperatur und Normaldruck hydriert. Nach Abfiltrieren vom Katalysator wird eingedampft. Der Rückstand wird über Kieselgel mit Methylenchlorid/Ethanol 6:1 chromatographiert. Man erhält 30 mg 3-(6-Methyl-2-vinyl-8α-ergolinyl)-1.1-diethylharnstoff nach Umkristallisation aus Ethanol/Hexan.
F.: 145-150 °C,
$[\alpha]_D$ = +63,5 ° (c = 0,22 % in Pyridin).

Beispiel 23

Man löst 800 mg 3-(2-Brom-1-(tert.butyl-dimethylsilyl)-6-methyl-8α-ergolinyl)-1.1-diethylharnstoff (1,5 mMol) in 75 ml wasserfreiem, frisch destilliertem Tetrahydrofuran unter Argonatmosphäre. Zu dieser Lösung fügt man 1 ml wasserfreies Tetramethylethylendiamin hinzu und kühlt auf -80 °C (Badtemperatur) ab. Dann wird mit 6,0 ml 1,4 m tert.-Butyllithiumlösung in Hexan (8,4 mMol) versetzt. Man erhält eine Lösung von 3-(1-tert.-Butyldimethylsilyl)-2-lithium-6-methyl-8α-ergolinyl)-1.1-diethylharnstoff, die 5 Stunden bei -70 °C gerührt wird. Nach Aufwärmen auf Raumtemperatur gibt man Wasser zu, schüttelt mit Methylenchlorid aus, trocknet die organische Phase mit Natriumsulfat und dampft ein. Der Rückstand wird an Kieselgel mit Methylenchlorid und Methanol chromatographiert. Man isoliert 265 mg 3-(2-tert.-Butyldimethylsilyl)-6-methyl-8α-ergolinyl)-1.1-diethylharnstoff (40 % d.Th.)
$[\alpha]_D$ = +30 ° (0,5 % in Chloroform).

Beispiel 24

Aus 1-(tert.-Butyl-dimethylsilyl)-2-brom-8.9-didehydro-6.8-dimethylergolin bereitet man sich mit tert.-Butyllithium wie in Beispiel 23 beschrieben die 2-Lithiumverbindung in wasserfreiem Tetrahydrofuran. Diese Lösung läßt man bei -70 °C 5 Stunden rühren, arbeitet dann in der üblichen Weise auf und chromatographiert.
Ausbeute: 65 % 2-(tert.-Butyl-dimethylsilyl)-8.9-didehydro-6.8-dimethyl-ergolin.
$[\alpha]_D$ = -152 ° (0,5 % in Chloroform).

In gleicher Weise werden die folgenden 2-Silylverbindungen hergestellt:
3-(2-(tert.-Butyl-dimethylsilyl)-6-methyl-8α-ergolinyl)-1.1-diethylthioharnstoff,
3-(2-(tert.-Butyl-dimethylsilyl)-9.10-didehydro-6-methyl-8α-ergolinyl)-1.1-diethylharnstoff, Ausbeute: 31 %;
$[\alpha]_D$ = + 305 ° (0,5 % in Chloroform).

8.9-Didehydro-6.8-dimethyl-2-trimethylsilyl-ergolin,
Ausbeute: 60 %; $[\alpha]_D$ = -177 ° (0,5 % in Chloroform),
1.1-Diethyl-3-(6-methyl-2-trimethylsilyl-8α-ergolinyl)-harnstoff, Ausbeute: 59 %; $[\alpha]_D$ = +30 ° (0,5 % in Chloroform).
3-(9.10-Didehydro-6-methyl-2-trimethylsilyl-8α-ergolinyl)-1.1-diethylharnstoff, Ausbeute: 37 %; $[\alpha]_D$ = 317 ° (0,5 % in Chloroform).
3-(6-n-Propyl-2-trimethylsilyl-8α-ergolinyl)-1.1-diethylharnstoff,
3-(2-tert.-Butyl-dimethylsilyl -6-methyl-8β-ergolinyl)-1.1-diethylharnstoff.

Beispiel 25

Zu einer Lösung von 6,8 g 1.1-Diethyl-3-(6-methyl-8α-ergolinyl)-harnstoff in 200 ml Chloroform gibt man bei Raumtemperatur unter Argon nacheinander 7 ml Ameisensäureethylester und 3,6 ml (44 mMol) Ethandithiol. Danach tropft man langsam 8,8 ml (80 mMol) Titan-IV-chlorid in 100 ml Chloroform gelöst dazu. Es werden 20 Stunden bei Raumtemperatur nachgerührt. Obwohl das Ausgangsmaterial zu diesem Zeitpunkt noch nicht vollständig umgesetzt ist, wird das Reaktionsgemisch aufgearbeitet, um eine weitere Entstehung der di-substituierten Verbindung zu vermeiden. Hierzu wird das Reaktionsgemisch im Eisbad gekühlt und nacheinander tropfenweise mit 50 ml Methanol und 40 ml Wasser versetzt. Dann wird die Mischung mit 25 %iger Ammoniaklösung alkalisiert und mit Methylenchlorid extrahiert. Die organischen Phasen wäscht man mit Wasser und trocknet sie über Magnesiumsulfat. Der eingedampfte Rückstand wird in der Siedehitze aus Methanol/Essigester kristallisiert. Man erhält 3,8 g 1.1-Diethyl-3-[2-(1.3-dithiolan-2-yl)-6-methyl-8α-ergolinyl]-harnstoff
(42 % Ausbeute).
$[\alpha]_D$ = +29 ° (0,5 % in $CH_3OH$).

Beispiel 26

7,5 ml einer Raney-Nickel-Suspension werden 4mal mit je 30 ml Methanol gewaschen. Dann gibt man 15 ml Methanol und anschließend eine Lösung aus 670 mg (1,5 mMol) 1.1-Diethyl-3-[2-(1.3-dithiolan-2-yl)-6-methyl-8α-ergolinyl]-harnstoff in 15 ml Methanol dazu. Man rührt 3 Stunden bei Raumtemperatur nach und gibt nochmals 7,5 ml einer Raney-Nickel-Suspension zu, die man vorher wie oben 4mal mit je 30 ml Methanol gewaschen hat. Nach weiteren 2 Stunden Rühren bei Raumtemperatur wird der Katalysator über eine Kieselgelsäule abfiltriert und mit Methanol gründlich gewaschen. Das Filtrat wird eingedampft und der Rückstand aus Methanol/Essigester kristallisiert. Man erhält 170 mg 1.1-Diethyl-3-(2.6-dimethyl-8α-ergolinyl)-harnstoff (32 % Ausbeute).
$[\alpha]_D$ = +12° (0,5 % in $CHCl_3$)

Beispiel 27

2,05 g (5 mMol) 1.1-Diethyl-3-[2-(1.3-dithiolan-2-yl)-6-methyl-8α-ergolinyl]-harnstoff werden in 46 ml Chloroform unter Argon gelöst. Dann gibt man 3,5 g Kieselgel und unter kräftigem Rühren tropfenweise 3,5 ml Wasser dazu. Während eines Zeitraumes von 30 Minuten tropft man eine Lösung aus 1,18 ml Sulfurylchlorid in 30 ml Chloroform zu. Nach 3 Stunden Rühren bei Raumtemperatur werden 7,5 g Kaliumcarbonat zugegeben und 20 Minuten kräftig gerührt. Der Niederschlag wird abfiltriert und mit Chloroform nachgewaschen. Den Niederschlag befeuchtet man mit Ethanol und gibt ihn in 300 ml gesättigte Kochsalzlösung, welche man dann mit Chloroform mehrmals extrahiert. Die Chloroformextrakte und das Chloroformfiltrat werden gemeinsam über Magnesiumsulfat getrocknet und eingedampft. Die anschließende Kristallisation erfolgt aus Essigester. Es werden 0,93 g 1.1-Diethyl-3-(6-methyl-2-formyl-8α-ergolinyl)-harnstoff (54 % Ausbeute) isoliert.
$[\alpha]_D$ = +78 ° (0,5 % in Pyridin).

Beispiel 28

320 mg (8 mMol) Lithiumaluminiumhydrid suspendiert man unter Argon in 20 ml absolutem frisch destillierten Tetrahydrofuran. Bei Raumtemperatur wird eine Lösung aus 1,40 g (4 mMol) 1.1-Diethyl-3-(6-methyl-2-formyl-8α-ergolinyl)-harnstoff in 40 ml frisch destilliertem,absolutem Tetrahydrofuran zugetropft. Anschließend rührt man 1 1/4 Stunden bei Raumtemperatur. Der Ansatz wird dann im Eisbad abgekühlt und mit 20 ml 1n Salzsäure versetzt. Man gibt 20 ml 2n-Weinsäure dazu und überschichtet den Ansatz mit

Essigester. Zur Neutralisation werden 80 ml in Ammoniaklösung zugetropft. Die Essigesterphase wird abgetrennt und die wässrige Phase mit Essigester nachextrahiert. Die vereinigten Essigesterphasen trocknet man über Natriumsulfat. Das eingeengte Rohprodukt wird an Kieselgel mit Methylenchlorid/Methanol 95:5 unter Druck chromatographiert. Das Rohprodukt (1,09 g) wird aus Essigester kristallisiert, wobei man 0,94 g 1.1-Diethyl-3-(6-methyl-2-hydroxymethyl-8α-ergolinyl)-harnstoff
(65 % Ausbeute) erhält.
$[\alpha]_D$ = +38 ° (0,5 % in Pyridin).

Beispiel 29

11,5 g (0,03 Mol) 3-(9.10-Didehydro-6-methyl-8α-ergolinyl)-1.1-diethyl-harnstoff werden in 300 ml Chloroform unter Argon gelöst. Bei Raumtemperatur gibt man nacheinander 105 ml Ameisensäureethylester und 5,4 ml Ethandithiol zu. Anschließend werden 13,2 ml Titan(IV)-chlorid in 150 ml Chloroform zugetropft. Man rührt 3 Tage bei Raumtemperatur. Zur Aufarbeitung kühlt man den Ansatz im Eisbad und tropft nacheinander 75 ml Methanol und 600 ml Wasser zu. Die Lösung wird mit 60 ml 25 %iger Ammoniaklösung alkalisiert und mit Methylenchlorid extrahiert. Die organischen Phasen wäscht man mit Wasser und trocknet sie über Magnesiumsulfat. Der eingeengte Rückstand wird unter Druck an Kieselgel mit Essigester/Methanol 95:5 chromatographiert, wobei 1,7 g 3-[9.10-Didehydro-2-(1.3-dithiolan-2-yl)-6-methyl-8α-ergolinyl]-1.1-diethylharnstoff (12 % Ausbeute) isoliert werden.

Beispiel 30

1,10 g (2,5 mMol) 3-[9.10-Didehydro-2-(1.3-dithiolan-2-yl)-6-methyl-8α-ergolinyl]-1.1-diethylharnstoff werden in 25 ml Chloroform unter Argon gelöst und mit 1,9 g Kieselgel der Korngröße 0,063-0,2 mm versetzt. Unter kräftigem Rühren tropft man 2 ml destilliertes Wasser dazu. Anschließend wird im Zeitraum von 15 Minuten eine Lösung von o,5 ml (0,6 mMol) Sulfurylchlorid in 13 ml Chloroform zugetropft und 2 Stunden bei Raumtemperatur gerührt. Zur Aufarbeitung gibt man 3 g Kaliumcarbonat zu und rührt 20 Minuten kräftig nach. Den Niederschlag filtriert man ab und wäscht ihn mit Methylenchlorid. Mit Ethanol wird der Niederschlag befeuchtet und in 250 ml gesättigte Kochsalzlösung aufgeschlämmt. Diese Mischung wird mit Methylenchlorid mehrmals extrahiert. Die Extrakte werden zusammen mit dem Methylenchloridfiltrat über Magnesiumsulfat getrocknet und eingeengt. Das Rohprodukt chromatographiert man unter Druck an Kieselgel mit einem Chloroform/Methanol-Gemisch und kristallisiert die Hauptfraktion von 448 mg aus Diisopropylether um. Dabei erhält man 225 mg (3-(9.10-Didehydro-2-formyl-6-methyl-8α-ergolinyl)-1.1-diethylharnstoff (25 % Ausbeute).
$[\alpha]_D$ = +398 ° (0,5 % in $CHCl_3$).

Beispiel 31

Zu einer Suspension von 52 mg (1,45 mMol) Lithiumaluminiumhydrid in 3 ml absolutem, frisch destilliertem Tetrahydrofuran unter Argon tropft man bei Raumtemperatur eine Lösung aus 244 mg (0,67 mMol) 3-(9.10-Didehydro-2-formyl-6-methyl-8α-ergolinyl)-1.1-diethyl-harnstoff in 25 ml absolutem, frisch destilliertem Tetrahydrofuran. 1 Stunde wird bei Raumtemperatur gerührt. Der Ansatz wird im Eisbad gekühlt und mit 3 ml 1n Salzsäure und 3 ml 2n-Weinsäure nacheinander versetzt. Die Mischung wird mit Essigester überschichtet und mit 12 ml 1n Anmoniaklösung alkalisch gestellt. Die Essigesterphase trennt man ab und extrahiert die wässrige Phase noch 2mal mit Essigester. Die vereinigten Essigesterphasen werden über Natriumsulfat getrocknet und eingeengt. Die Kristallisation erfolgt aus Essigester, wobei 161 mg 3-(9,10-Didehydro-2-hydroxymethyl-6-methyl-8α-ergolinyl)-1.1-diethy--harnstoff (65% Ausbeute) erhalten werden.
$[\alpha]_D$ = + 328 ° (0,5 % in Pyridin).

Beispiel 32

Analog Beispiel 6 erhält man aus 3-(2-Brom-1.tert.-butoxy-dimethylsilyl-6-methyl-8α-ergolinyl)-1.1-diethylthioharnstoff mit tert.-Butyllithium die entsprechende Lithiumverbindung und daraus mit Dimethyldisulfid den 1.1-Diethyl-3-(6-methyl-2-methylthio-8α-ergolinyl)-thioharnstoff.
Ausbeute: 38 %, $[\alpha]_D$ = + 54 ° (0,5 % in Chloroform).

Beispiel 33

a) Zu 4 ml absolutem, frisch destilliertem Toluol gibt man unter Argon 0,3 ml (1,5 mMol) destilliertes Hexamethyldisilazan und kühlt die Mischung auf 0 °C ab. Dann werden 0,85 ml (1,4 mMol) 15 %iges Butyllithium/Hexan zugetropft und 15 Minuten bei 0 °C nachgerührt. Dazu gibt man 531 mg (1 mMol) 3-(2-Brom-1-(tert.-butyl-dimethylsilyl)-9.10-didehydro-6-methyl-8α-ergolinyl)-1.1-diethylharnstoff in 50 ml absolutem, frisch destilliertem Toluol und rührt wieder 15 Minuten bei 0 °C nach. Nach der Zugabe von 1 ml destilliertem Tetramethylethylendiamin wird der Ansatz auf -90 °C abgekühlt. Bei dieser Temperatur gibt man 5 ml (7 mMol) 1,4 ml tert.-Butyllithium zu und rührt 2 Minuten nach, bevor man 0,6 ml (5,4 mMol) Trifluormethansulfonsäure-methylester zugbit. Nach 1 1/2 Stunden Rühren bei -70 °C wird zur Aufarbeitung Wasser zugegeben. Die Extraktion erfolgt mit Esgigester. Die organischen Phasen werden mit gesättigter Natriumhydrogencarbonatlösung und Wasser gewaschen. Die vereinigten Essigesterphasen werden über Natriumsulfat getrocknet und eingeengt. Nach Kristallisation aus Methanol erhält man 128 mg 3-[1-tert.-Butyl-dimethylsilyl)-9.10-didehydro-2.6-dimethyl-8α-ergolinyl]-1.1-diethyl-harnstoff (27 % Ausbeute).
$[\alpha]_D$ = + 252,1 ° (0,5 % in $CHCl_3$).
b) 183 mg (0,39 mMol) 3-[1-(tert.-Butyl-dimethylsilyl)-9.10-didehydro-2.6-dimethyl-8α-ergolinyl]-1.1-diethyl-harnstoff werden in 5 ml Methanol unter Zugabe von 2 ml Tetrahydrofuran unter Argon gelöst und mit 1 ml 14N Kalilauge bei Raumtemperatur 17 Stunden gerührt. Zur Aufarbeitung verdünnt man den Ansatz mit gesättigter Kochsalzlösung und extrahiert mit Methylenchlorid. Die Methylenchloridextrakte werden mit gesättigter Kochsalzlösung und Wasser gewaschen und über Natriumsulfat getrocknet. Das Rohprodukt von 128 mg wird chromatographiert und als weinsaures Salz kristallisiert. Man erhält 94 mg
3-(9,10-Didehydro-2,6-dimethyl-8α-ergolinyl)-1,1-diethylharnstoff
(67 % d. Th.).
$[\alpha]_D$ = +222 ° (0,1 % in Pyridin).

In analoger Weise wird durch Ersatz des Trifluormethansulfonsäuremethylesters durch den -Ethylester der 3-(9.10-Didehydro-2-ethyl-6-methyl-8α-ergolinyl)-1.1-diethylharnstoff erhalten und als weinsaures Salz kristallisiert.
Ausbeute: 21 %; $[\alpha]_D$ = +195 ° (0,1 % in Pyridin).

Beim Ersatz des Trifluormethansulfonsäureesters durch p-Toluolsulfonsäurethioester (ethyl, n-propyl und isopropyl) werden die folgenden Verbindungen erhalten:
3-(9.10-Didehydro-2-ethylthio-6-methyl-8α-ergolinyl)-1.1-diethylharnstoff.
Ausbeute: 26 %; $[\alpha]_D$ = +299 ° (0,5 % in Chloroform).
3-(9.10-Didehydro-6-methyl-2-n-propylthio-8α-ergolinyl)-1.1-diethyl-harnstoff.
Ausbeute: 38 %; $[\alpha]_D$ = 284 ° (0,5 % in Chloroform).
3-(9.10-Didehydro-6-methyl-2-iso-propylthio-8α-ergolinyl)-1.1-diethyl-harnstoff.
Ausbeute: 21 %; $[\alpha]_D$ = +286 ° (0,5 % in Chloroform).

Verwendet man als Ausgangsmaterial 3-(2-Brom-1-(tert.-butyl-dimethylsilyl)-6-methyl-8α-ergolinyl)-1.1-diethyl harnstoff so erhält man mit Trifluormethansulfonsäureethylester den 3-(2-Ethyl-6-methyl-8α-ergolinyl)-1.1-diethyl-harnstoff.
Ausbeute: 29 %;
mit p-Toluolsulfonsäure-thioethylester den 3-(2-Ethylthio-6-methyl-8α-ergolinyl)-1.1-diethylharnstoff.
Ausbeute: 78 %; $[\alpha]_D$ = +5 ° (0,5 % in Chloroform);
mit p-Toluolsulfonsäure-thio-n-propylester den 1.1-Diethyl-3-(6-methyl-2-n-propylthio-8α-ergolinyl)-harnstoff.
Ausbeute: 75 %; $[\alpha]_D$ = +4 ° (0,5 % in Chloroform);
mit p-Toluolsulfonsäure-thio-iso-propylester den 1.1-Diethyl-3-(6-methyl-2-iso-propylthio-8α-ergolinyl)-harnstoff. Ausbeute: 35 %; $[\alpha]_D$ = + 4 ° (0,5 % in Chloroform)

Beispiel 34

1,4 g (3 mMol) 3-(2-Jod-6-methyl-8α-ergolinyl)-1.1-diethylharnstoff, 37,5 mg (0,123 mMol) Tri-o-tolylphosphin, 25,1 mg (0,032 mMol) trans-Dichlor-bis-(tri-o-tolylphosphin)-palladium(II) und 1,13 ml (7,8 mMol) Vinyltrimethylsilan werden in 7,5 ml Trimethylformamid und 7,5 ml Triethylamin gelöst und nach Spülen mit Argon im Autoklaven 3 Stunden auf 100 °C erhitzt. Nach dem Einengen der Reaktionslösung unter vermindertem Druck wird in Dichlormethan aufgenommen und mit gesättigter Natriumchlorid-Lösung gewaschen. Nach Trocknen über Magnesiumsulfat wird die organische Phase eingeengt. Aus dem Rohprodukt erhält man nach Säulenchromatographie über Kieselgel mit Dichlormethan/Ethanol 10:1 als Elutionsmittel und Umkristallisation aus Ethanol/Petrolether 400 mg 3-[6-Methyl-2-(trans-2-trimethylsilyl-1-ethenyl)-8α-ergolinyl]-1.1-diethylharnstoff (30,2 %). F.: 194-197 °C.
$[\alpha]_D$ = +89,0 ° (c = 0,225 % in Pyridin);
und
563 mg 3-(2-Ethenyl-6-methyl-8α-ergolinyl)-1.1-diethylharnstoff (51,2 %). F.: 145-150 °C.
$[\alpha]_D$ = +63,5 ° (c = 0,23 % in Pyridin).

**Patentansprüche**
**Patentansprüche für folgende Vertragsstaaten : BE, CH, DE, FR, GB, IT, LI, LU, NL, SE**

**1.** In 2-Stellung substituierte Ergolin-Derivate der allgemeinen Formel I

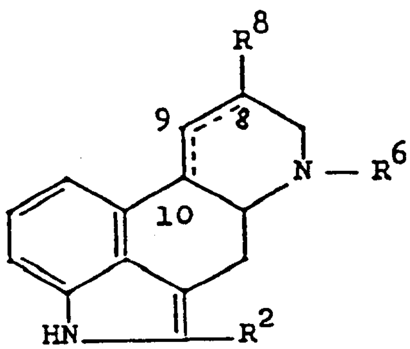
(I),

worin
$C_8$---$C_9$ und $C_9$---$C_{10}$ eine CC-Einfach- oder eine C=C-Doppelbindung, jedoch keine kumulierte Doppelbindung bedeutet und der Substituent in 8-Stellung α- oder β-ständig ist, wenn $C_8$---$C_9$ eine CC-Einfachbindung bedeutet,
$R^2$ CN,SR, SOR,

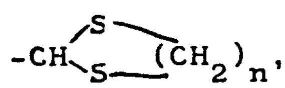

wobei n = 2 oder 3 ist,

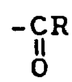

und -C(HOH)R mit R in der Bedeutung von
H oder $C_{1-4}$-Alkyl,
die Gruppierung COR' und CSR'
(R' = OH, $OC_{1-4}$-Alkyl, Benzyloxy, $NH_2$ oder NHR''),
die Gruppierung $CH_2$-$CH_2$-$CO_2R''$
(R'' = $C_{1-4}$-Alkyl),
die Gruppierung C≡C-R''' und HC=CH-R'''
(R''' = Wasserstoff, $C_{1-4}$-Alkyl, Phenyl, $CH_2OH$, $CR''_2OH$,

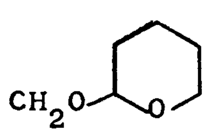
,

$CO_2R''$, $CH_2NR''_2$, $SiMe_2R''$) und
$C_{1-3}$-Alkyl, das mit OH oder Phenyl substituiert sein kann,

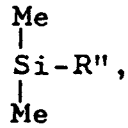

wobei R'' $C_{1-4}$-Alkyl
bedeutet,
$R^6$ $C_{1-4}$-Alkyl und
$R^8$ Methyl oder die Gruppierung NH-CO-$NEt_2$ oder NH-CS-$NEt_2$ darstellen, und deren Salze, wobei $C_8$---$C_9$ eine Doppelbindung darstellt, falls $R^8$ $CH_3$ ist.

**2.** 3-(9.10-Didehydro-6-methyl-2-methylthio-8α-ergolinyl)-1.1-diethylharnstoff und dessen Tartrat.
3-(6-Methyl-2-methylthio-8α-ergolinyl)-1.1-diethylharnstoff und dessen Tartrat.
1.1-Diethyl-(6-methyl-2-propyl-8α-ergolinyl)-harnstoff
1.1-Diethyl-(6-methyl-2-isopropyl-8α-ergolinyl)-harnstoff
8α-(3.3-Diethylureido)-2.6-dimethyl-ergolin
1.1-Diethyl-(6-methyl-2-ethinyl-8α-ergolinyl)-harnstoff
1.1-Diethyl-(6-methyl-2-ethylthio-8α-ergolinyl)-harnstoffnach Anspruch 1.

**3.** 3-(2-Ethinyl-1.6-dimethyl-8α-ergolinyl)-1.1-diethylharnstoff

**4.** Verfahren zur Herstellung von Verbindungen der Formel IA und deren Salze

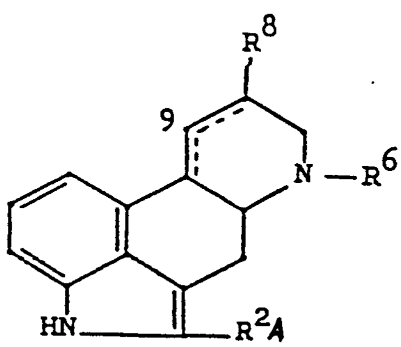

IA

worin
$C_8$...$C_9$ und $C_9$...$C_{10}$ eine Einfach- oder Doppelbindung, jedoch keine kumulierte Doppelbindung bedeuten und der Substituent in 8-Stellung α- oder β-ständig ist, wenn $C_8$...$C_9$ eine Einfachbindung bedeutet und $C_8$...$C_9$ eine Doppelbindung ist, wenn $R^8$ $CH_3$ bedeutet,
$R^6$ $C_{1-4}$-Alkyl,
$R^8$ Methyl, NH-CO-$NEt_2$ oder NH-CS-$NEt_2$ und
$R^{2A}$ CN, SR, SOR, -CO-R oder -CHOH-R mit R in der Bedeutung von H oder $C_{1-4}$-Alkyl ist,
dadurch gekennzeichnet, daß man eine Verbindung der Formel II

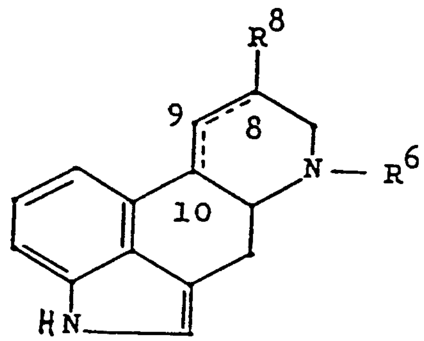

(II),

worin

$C_8 \dots C_9$, $C_9 \dots C_{10}$, $R^6$ und $R^8$ die oben angegebene Bedeutung haben, mit einem elektrophilen Reagenz aus der Gruppe Chlorsulfonylisocyanat, Alkylthio-sulfoniumfluorborat oder Acylchlorid in Gegenwart einer Lewis-Säure umsetzt, gewünschtenfalls anschließend eine Carbonylgruppe am 2-Substituenten mit einem komplexen Metallhydrid reduziert oder eine Alkylthiogruppe mit Periodat oxidiert oder die Säureadditionssalze bildet.

**5.** Verfahren zur Herstellung von Verbindungen der Formel IB und deren Salze

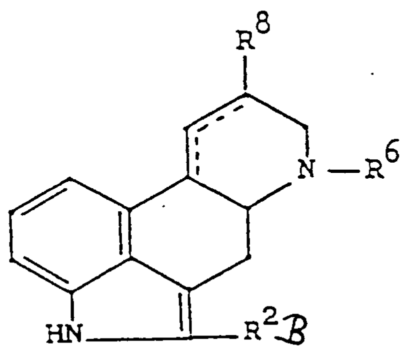

IB

worin

$C_8 \dots C_9$ und $C_9 \dots C_{10}$ eine Einfach- oder Doppelbindung, jedoch keine kumulierte Doppelbindung bedeuten und der Substituent in 8-Stellung $\alpha$- oder $\beta$-ständig ist, wenn $C_8 \dots C_9$ eine Einfachbindung bedeutet und $C_8 \dots C_9$ eine Doppelbindung ist, wenn $R^8$ $CH_3$ bedeutet,

$R^6$ $C_{1-4}$-Alkyl,

$R^8$ Methyl, NH-CO-$NEt_2$ oder NH-CS-$NEt_2$ und

$R^{2B}$ $C_{1-3}$-Alkyl, Hydroxyethyl, $C_{2-3}$-Alkenyl, -COOH -CONHR", -CSNHR", -COOR", -S-$C_{1-4}$-Alkyl oder - $SiMe_2C_{1-4}$-Alkyl bedeutet und R" $C_{1-4}$-Alkyl ist, dadurch gekennzeichnet, daß man eine Verbindung der Formel III

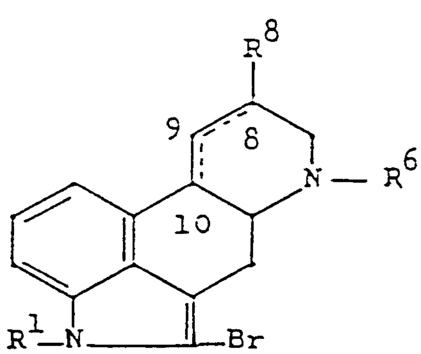

(III),

worin
$C_8...C_9$, $C_9...C_{10}$, $R^6$ und $R^8$ die oben angegebene Bedeutung haben und
$R^1$ eine Schutzgruppe darstellt, mit Lithiumalkyl oder mit Lithiumphenyl bei Temperaturen unter 0 °C umsetzt und das so erhaltene 2-Li-Ergolin-Derivat der allgemeinen Formel IV

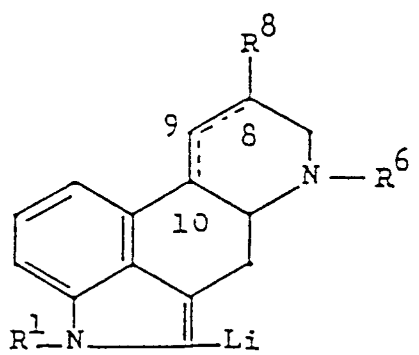

(IV),

worin
$C_8...C_9$, $C_9...C_{10}$, $R^1$, $R^6$ und $R^8$ die oben angegebene Bedeutung haben, mit einem elektrophilen Reagenz aus der Gruppe Kohlendioxid, Ethylenoxid, $C_{1-4}$-Alkylisocyanat, $C_{1-4}$-Alkylthioisocyanat, $C_{1-4}$-Alkyldimethylchlorsilan, $C_{1-3}$-Alkyl- und $C_{2-3}$-Alkenylbromid bzw. -jodid oder $C_{1-4}$Alkyldisulfide bei tiefen Temperaturen von -110 bis -50 °C in einem inerten Lösungsmittel umsetzt und gewünschtenfalls eine freie 2-Carboxylfunktion mit einem aliphatischen Alkohol der Formel R"OH verestert oder die Schutzgruppe $R^1$ abspaltet oder die Säureadditionssalze bildet.

**6.** Verfahren zur Herstellung der Verbindungen der Formel IC und deren Salze

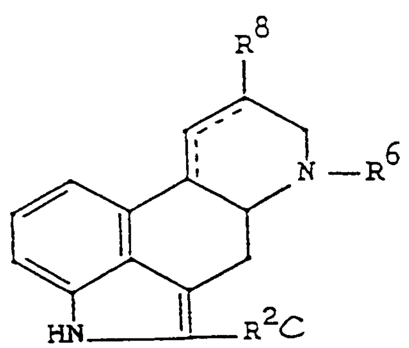

IC

worin
$C_8...C_9$ und $C_9...C_{10}$ eine Einfach- oder Doppelbindung, jedoch keine kumulierte Doppelbindung bedeuten und der Substituent in 8-Stellung $\alpha$- oder $\beta$-ständig ist, wenn $C_8...C_9$ eine Einfachbindung bedeutet und $C_8...C_9$ eine Doppelbindung ist, wenn $R^8$ $CH_3$ bedeutet,
$R^6$ $C_{1-4}$-Alkyl,
$R^8$ Methyl, NH-CO-$NEt_2$ oder NH-CS-$NEt_2$ und
$R^{2C}$ die Gruppierung COOH, CO-O-Benzyl, CO-$NH_2$ oder
$CH_2$-$CH_2$-$CO_2$R" (R" = $C_{1-4}$-Alkyl),
die Gruppierung C≡C-R''' oder HC=CH-R'''
(R''' = Wasserstoff, $C_{1-4}$-Alkyl, Phenyl, $CH_2OH$, $CR''_2OH$,

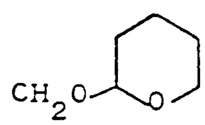,

$CO_2R''$, $CH_2NR''_2$, $SiMe_2R''$)
$C_{2-3}$-Alkyl, mit Phenyl substituiertes Ethyl oder mit OH substituiertes Propyl bedeutet, dadurch gekennzeichnet, daß man eine Verbindung der Formel V

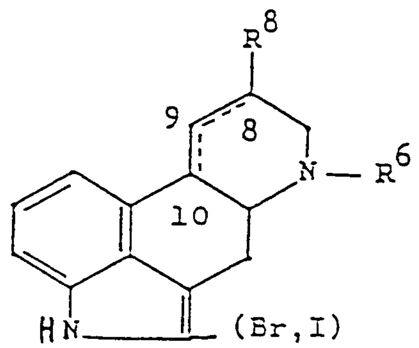

(V),

worin
$C_8\text{---}C_9$ , $C_9\text{---}C_{10}$, $R^6$ und $R^8$ die oben angegebene Bedeutung haben, mit einem elektrophilen Reagenz aus der Gruppe Benzylalkohol/Kohlenmonoxid, Acrylester $CH_2=CH\text{-}CO_2R''$ oder Ethinylderivat $CH\equiv C\text{-}R'''$, worin R'' und R''' die oben angegebene Bedeutung haben, in Gegenwart eines Palladium-Katalysators und eines sekundären oder tertiären Amins ohne Lösungsmittel oder in einem aprotischen, mit Wasser mischbaren Lösungsmittel bei Temperaturen oberhalb Raumtemperatur im Bereich von 40 °C bis zur Siedetemperatur des Reaktionsgemisches reagieren läßt, gewünschtenfalls anschließend die 2-Benzylgruppe mit Palladium unter Bildung der Carboxylverbindung hydriert oder mit Ammoniak zur 2-Carbonsäureamidgruppe umsetzt oder eine exocyclische Mehrfachbindung mit Raney-Nickel oder Palladium auf einem Träger zur CC-Einfachbindung oder Doppelbindung hydriert oder eine $SiMe_2R''$-Schutzgruppe mit einer Base oder eine Tetrahydropyranyl-Schutzgruppe mit Pyridinium-p-toluolsulfonat abspaltet oder die Säureadditionssalze bildet.

**7.** Verfahren zur Herstellung von Verbindungen der Formel ID und deren Salze

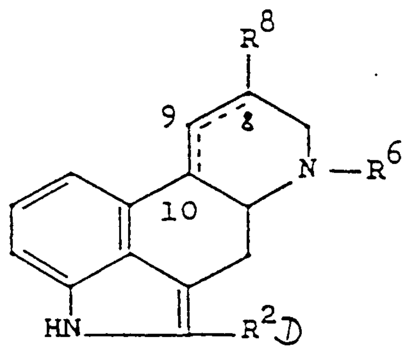

ID.

worin
$C_8...C_9$ und $C_9...C_{10}$ eine Einfach- oder Doppelbindung, jedoch keine kumulierte Doppelbindung bedeuten und der Substituent in 8-Stellung α- oder β-ständig ist, wenn $C_8...C_9$ eine Einfachbindung bedeutet und $C_8...C_9$ eine Doppelbindung ist, wenn $R^8$ $CH_3$ bedeutet,
$R^6$ $C_{1-4}$-Alkyl,
$R^8$ Methyl, NH-CO-$NEt_2$ oder NH-CS-$NEt_2$ und

$R^{2D}$ $SiMe_2$-$C_{1-4}$-Alkyl ist,
dadurch gekennzeichnet, daß man eine Verbindung der Formel VI

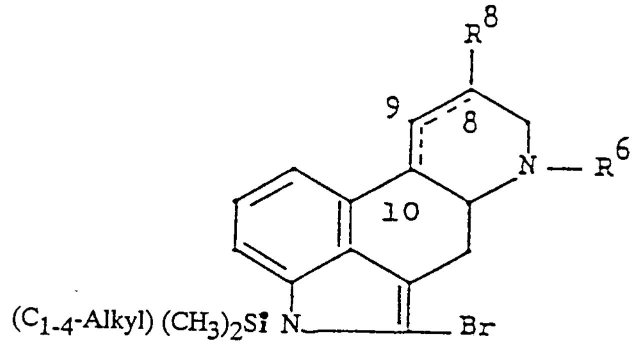

(VI),

worin
$C_8$---$C_9$, $C_9$---$C_{10}$, $R^6$ und $R^8$ die oben angegebene Bedeutung haben, mit einem Lithiumalkyl bei Temperaturen unten 0 °C gegebenenfalls in Gegenwart eines tert.-Amins umsetzt,
und gewünschtenfalls die Säureadditionssalze bildet.

**8.** Verwendung der Verbindungen nach den Ansprüchen 1 bis 3 zur Herstellung eines Arzneimittels.

**9.** Verfahren zur Herstellung eines Arzneimittels dadurch gekennzeichnet, daß man eine der Verbindungen nach den Ansprüchen 1 bis 3 mit üblichen Träger- und Hilfsstoffen mischt.

**Patentansprüche für folgenden Vertragsstaat : AT**

**1.** Verfahren zur Herstellung von Verbindungen der Formel IA und deren Salze

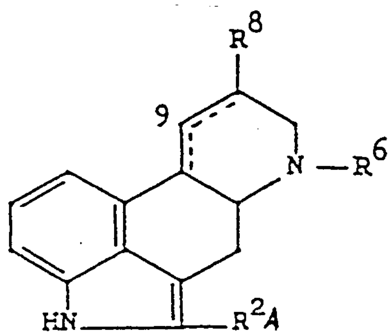

IA

worin
$C_8$...$C_9$ und $C_9$...$C_{10}$ eine Einfach- oder Doppelbindung, jedoch keine kumulierte Doppelbindung bedeuten und der Substituent in 8-Stellung $\alpha$- oder $\beta$-ständig ist, wenn $C_8$...$C_9$ eine Einfachbindung bedeutet und $C_8$...$C_9$ eine Doppelbindung ist, wenn $R^8$ $CH_3$ bedeutet,
$R^6$ $C_{1-4}$-Alkyl,
$R^8$ Methyl, NH-CO-$NEt_2$ oder NH-CS-$NEt_2$ und
$R^{2A}$ CN, SR, SOR, -CO-R oder -CHOH-R mit R in der Bedeutung von H oder $C_{1-4}$-Alkyl ist,
dadurch gekennzeichnet, daß man eine Verbindung der Formel II

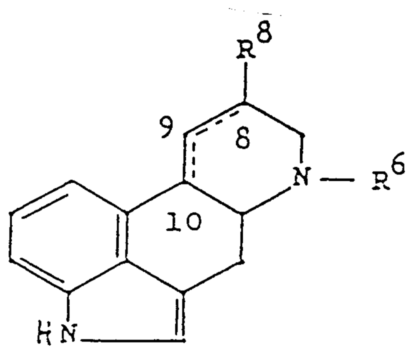

(II),

worin
$C_8...C_9$, $C_9...C_{10}$, $R^6$ und $R^8$ die oben angegebene Bedeutung haben,
mit einem elektrophilen Reagenz aus der Gruppe Chlorsulfonylisocyanat, Alkylthio-sulfoniumfluorborat oder Acylchlorid in Gegenwart einer Lewis-Säure umsetzt, gewünschtenfalls anschließend eine Carbonylgruppe am 2-Substituenten mit einem komplexen Metallhydrid reduziert oder eine Alkylthiogruppe mit Periodat oxidiert oder die Säureadditionssalze bildet.

**2.** Verfahren zur Herstellung von Verbindungen der Formel IB und deren Salze

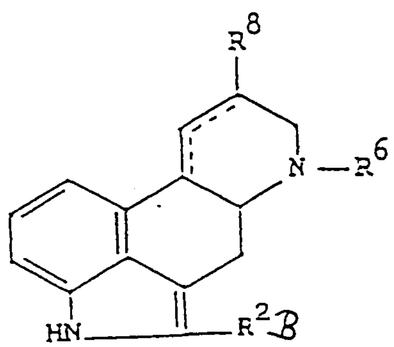

IB

worin
$C_8...C_9$ und $C_9...C_{10}$ eine Einfach- oder Doppelbindung, jedoch keine kumulierte Doppelbindung bedeuten und der Substituent in 8-Stellung $\alpha$- oder $\beta$-ständig ist, wenn $C_8...C_9$ eine Einfachbindung bedeutet und $C_8...C_9$ eine Doppelbindung ist, wenn $R^8$ $CH_3$ bedeutet,
$R^6$ $C_{1-4}$-Alkyl,
$R^8$ Methyl, NH-CO-$NEt_2$ oder NH-CS-$NEt_2$ und
$R^{2B}$ $C_{1-3}$-Alkyl, Hydroxyethyl, $C_{2-3}$-Alkenyl, -COOH -CONHR", -CSNHR", -COOR", -S-$C_{1-4}$-Alkyl oder - $SiMe_2C_{1-4}$-Alkyl bedeutet und R" $C_{1-4}$-Alkyl ist, dadurch gekennzeichnet, daß man eine Verbindung der Formel III

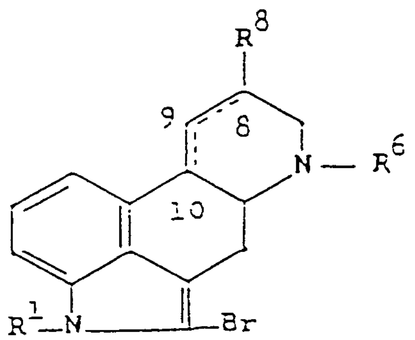

(III),

worin
$C_8...C_9$, $C_9...C_{10}$, $R^6$ und $R^8$ die oben angegebene Bedeutung haben und
$R^1$ eine Schutzgruppe darstellt, mit Lithiumalkyl oder mit Lithiumphenyl bei Temperaturen unter 0 °C umsetzt und das so erhaltene 2-Li-Ergolin-Derivat der allgemeinen Formel IV

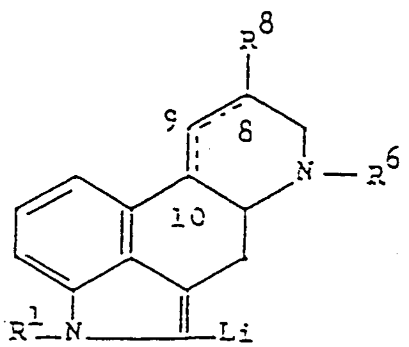

(IV),

worin
$C_8...C_9$, $C_9...C_{10}$, $R^1$, $R^6$ und $R^8$ die oben angegebene Bedeutung haben, mit einem elektrophilen Reagenz aus der Gruppe Kohlendioxid, Ethylenoxid, $C_{1-4}$-Alkylisocyanat, $C_{1-4}$-Alkylthioisocyanat, $C_{1-4}$-Alkyldimethylchlorsilan, $C_{1-3}$-Alkyl- und $C_{2-3}$-Alkenylbromid bzw. -jodid oder $C_{1-4}$Alkyldisulfide bei tiefen Temperaturen von -110 bis -50 °C in einem inerten Lösungsmittel umsetzt und gewünschtenfalls eine freie 2-Carboxylfunktion mit einem aliphatischen Alkohol der Formel R"OH verestert oder die Schutzgruppe $R^1$ abspaltet oder die Säureadditionssalze bildet.

**3.** Verfahren zur Herstellung der Verbindungen der Formel IC und deren Salze

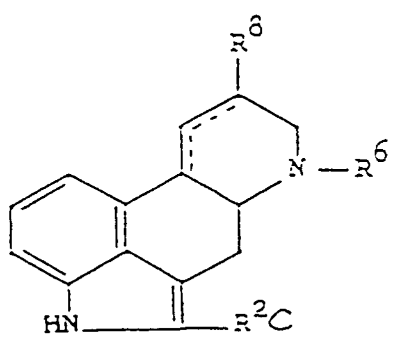

IC

worin
$C_8...C_9$ und $C_9...C_{10}$ eine Einfach- oder Doppelbindung, jedoch keine kumulierte Doppelbindung bedeuten und der Substituent in 8-Stellung α- oder β-ständig ist, wenn $C_8...C_9$ eine Einfachbindung bedeutet und $C_8...C_9$ eine Doppelbindung ist, wenn $R^8$ $CH_3$ bedeutet,
$R^6$ $C_{1-4}$-Alkyl,
$R^8$ Methyl, NH-CO-$NEt_2$ oder NH-CS-$NEt_2$ und
$R^{2C}$ die Gruppierung COOH, CO-O-Benzyl, CO-$NH_2$ oder
$CH_2$-$CH_2$-$CO_2$R" (R" = $C_{1-4}$-Alkyl),
die Gruppierung C≡C-R''' oder HC=CH-R'''
(R''' = Wasserstoff, $C_{1-4}$-Alkyl, Phenyl, $CH_2OH$, $CR''_2OH$,

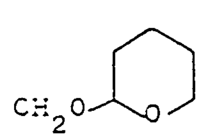

,

$CO_2R''$, $CH_2NR''_2$, $SiMe_2R''$)
$C_{2-3}$-Alkyl, mit Phenyl substituiertes Ethyl oder mit OH substituiertes Propyl bedeutet, dadurch gekennzeichnet, daß man eine Verbindung der Formel V

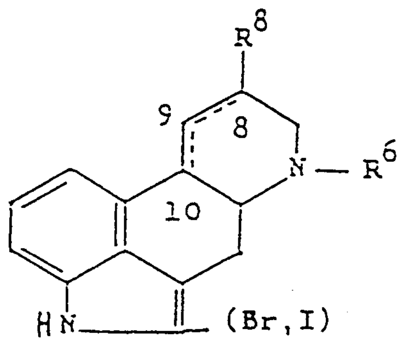

(V),

worin
$C_8$---$C_9$ , $C_9$---$C_{10}$, $R^6$ und $R^8$ die oben angegebene Bedeutung haben, mit einem elektrophilen Reagenz aus der Gruppe Benzylalkohol/Kohlenmonoxid, Acrylester $CH_2=CH-CO_2R''$ oder Ethinylderivat $CH{\equiv}C-R'''$ , worin R'' und R''' die oben angegebene Bedeutung haben, in Gegenwart eines Palladium-Katalysators und eines sekundären oder tertiären Amins ohne Lösungsmittel oder in einem aprotischen, mit Wasser mischbaren Lösungsmittel bei Temperaturen oberhalb Raumtemperatur im Bereich von 40 °C bis zur Siedetemperatur des Reaktionsgemisches reagieren läßt, gewünschtenfalls anschließend die 2-Benzylgruppe mit Palladium unter Bildung der Carboxylverbindung hydriert oder mit Ammoniak zur 2-Carbonsäureamidgruppe umsetzt oder eine exocyclische Mehrfachbindung mit Raney-Nickel oder Palladium auf einem Träger zur CC-Einfachbindung oder Doppelbindung hydriert oder eine $SiMe_2R''$-Schutzgruppe mit einer Base oder eine Tetrahydropyranyl-Schutzgruppe mit Pyridinium-p-toluolsulfonat abspaltet oder die Säureadditionssalze bildet.

**4.** Verfahren zur Herstellung von Verbindungen der Formel ID und deren Salze

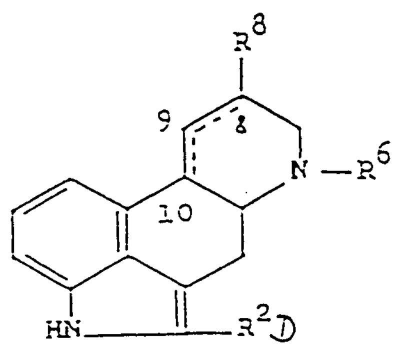

ID.

worin
$C_8$...$C_9$ und $C_9$...$C_{10}$ eine Einfach- oder Doppelbindung, jedoch keine kumulierte Doppelbindung bedeuten und der Substiuent in 8-Stellung α- oder β-ständig ist, wenn $C_8$...$C_9$ eine Einfachbindung bedeutet und $C_8$...$C_9$ eine Doppelbindung ist, wenn $R^8$ $CH_3$ bedeutet.
$R^6$ $C_{1-4}$-Alkyl,
$R^8$ Methyl, NH-CO-$NEt_2$, oder NH-CS-$NEt_2$ und
$R^{2D}$ $SiMe_2$-$C_{1-4}$-Alkyl ist,
dadurch gekennzeichnet, daß man eine Verbindung der Formel VI

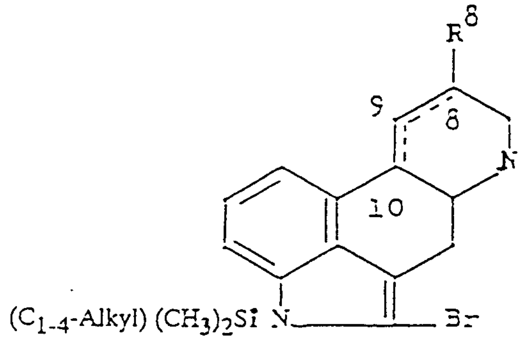

(VI),

worin $C_8$---$C_9$, $C_9$---$C_{10}$, $R^6$ und $R^8$ die oben angegebene Bedeutung haben, mit einem Lithiumalkyl bei Temperaturen unter 0 °C gegebenenfalls in Gegenwert eines tert.-Amins umsetzt, und gewünschtenfalls die Säureadditionssalze bildet.

**5.** Verfahren zur Herstellung der Verbindungen der Formel I

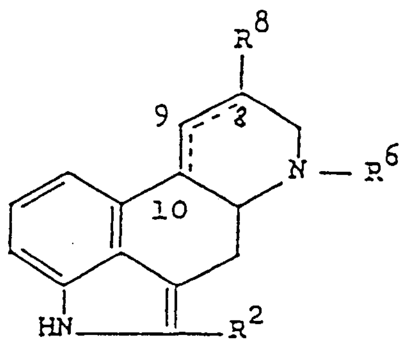

(I),

worin $C_8$---$C_9$ und $C_9$---$C_{10}$ eine CC-Einfach- oder eine C=C-Doppelbindung, jedoch keine kumulierte Doppelbindung bedeutet und der Substituent in 8-Stellung $\alpha$- oder $\beta$-ständig ist, wenn $C_8$---$C_9$ eine CC-Einfachbindung bedeutet,
$R^2$ CN,SR, SOR,

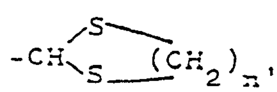

wobei n = 2 oder 3 ist,

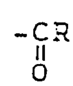

und -C(HOH)R mit R in der Bedeutung von
H oder $C_{1-4}$-Alkyl,
die Gruppierung COR' und CSR'
(R' = OH, $OC_{1-4}$-Alkyl, Benzyloxy, $NH_2$ oder NHR''),
die Gruppierung $CH_2$-$CH_2$-$CO_2$R''
(R'' = $C_{1-4}$-Alkyl),
die Gruppierung C≡C-R''' und HC=CH-R'''
(R''' = Wasserstoff, $C_{1-4}$-Alkyl, Phenyl, $CH_2OH$, $CR''_2OH$,

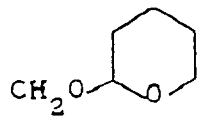,

$CO_2R''$, $CH_2NR''_2$, $SiMe_2R''$) und
$C_{1-3}$-Alkyl, das mit OH oder Phenyl substituiert sein kann,

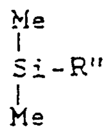,

wobei R'' $C_{1-4}$-Alkyl
bedeutet,
$R^6$ $C_{1-4}$-Alkyl und
$R^8$ Methyl oder die Gruppierung NH-CO-$NEt_2$ oder NH-CS-$NEt_2$ darstellen, und deren Salze, wobei $C_8\text{---}C_9$ eine Doppelbindung darstellt, falls $R^8$ $CH_3$ ist, durch Analogieverfahren gemäß den Ansprüchen 1-4.

**6.** Verfahren zur Herstellung eines Arzneimittels dadurch gekennzeichnet, daß man eine der nach den Ansprüchen 1-5 erhaltenen Verbindungen mit üblichen Träger- und Hilfsstoffen mischt.

**Claims**

**Claims for the following Contracting States : BE, CH, DE, FR, GB, IT, LI, LU, NL, SE**

**1.** Ergoline derivatives, substituted in the 2-position, of the general formula I

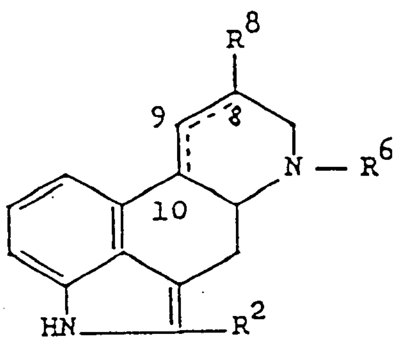

(I)

in which
$C_8\text{---}C_9$ and $C_9\text{---}C_{10}$ represent a CC single bond or a C=C double bond, but not a cumulative double bond, and the substituent in the 8-position is in the $\alpha$- or $\beta$-configuration when $C_8\text{---}C_9$ represents a CC single bond,
$R^2$ represents CN, SR, SOR,

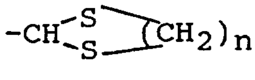,

wherein n = 2 or 3,

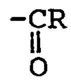

or -C(HOH)R, wherein R represents H or $C_1$-$C_4$-alkyl,
the group COR' or CSR' (R' = OH, $OC_{1-4}$-alkyl, benzyloxy, $NH_2$ or NHR"),
the group $CH_2$-$CH_2$-$CO_2$R" (R" = $C_{1-4}$-alkyl),
the group C≡C-R"' or HC=CH-R"' (R"' = hydrogen, $C_{1-4}$-alkyl, phenyl, $CH_2OH$, $CR"_2OH$,

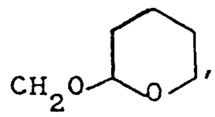

$CO_2$R", $CH_2NR"_2$, $SiMe_2$R"),
$C_{1-3}$-alkyl that may be substituted by OH or by phenyl, or

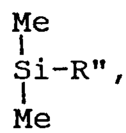

wherein R" represents $C_{1-4}$-alkyl,
$R^6$ represents $C_{1-4}$-alkyl, and
$R^8$ represents methyl or the group NH-CO-$NEt_2$ or NH-CS-$NEt_2$,
and their salts, $C_8$---$C_9$ representing a double bond when $R^8$ is $CH_3$.

**2.** 3-(9,10-didehydro-6-methyl-2-methylthio-8α-ergolinyl)-1,1-diethylurea and the tartrate thereof,
3-(6-methyl-2-methylthio-8α-ergolinyl)-1,1-diethylurea and the tartrate thereof,
1,1-diethyl-(6-methyl-2-propyl-8α-ergolinyl)-urea,
1,1-diethyl-(6-methyl-2-isopropyl-8α-ergolinyl)-urea,
8α-(3,3-diethylureido)-2,6-dimethyl-ergoline,
1,1-diethyl-(6-methyl-2-ethynyl-8α-ergolinyl)-urea,
1,1-diethyl-(6-methyl-2-ethylthio-8α-ergolinyl)-urea
according to claim 1.

**3.** 3-(2-ethynyl-1,6-dimethyl-8α-ergolinyl)-1,1-diethyl-urea.

**4.** Process for the preparation of compounds of formula IA and their salts

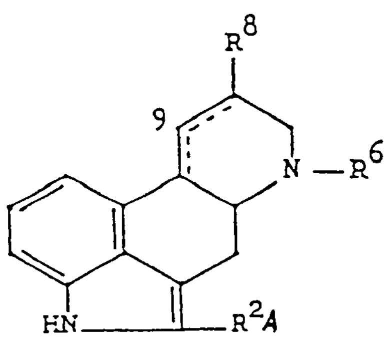

(IA)

in which
$C_8$---$C_9$ and $C_9$---$C_{10}$ represent a single bond or a double bond, but not a cumulative double bond, and the substituent in the 8-position is in the α- or β-configuration when $C_8$---$C_9$ represents a single bond,

and $C_8$---$C_9$ is a double bond when $R^8$ represents $CH_3$,
$R^6$ represents $C_{1-4}$-alkyl,
$R^8$ represents methyl, NH-CO-$NEt_2$ or NH-CS-$NEt_2$, and
$R^{2A}$ is CN, SR, SOR, -CO-R or -CHOH-R, wherein R represents H or $C_{1-4}$-alkyl,
characterised in that a compound of formula II

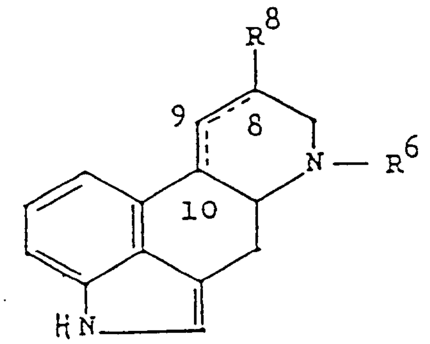

(II),

in which $C_8$---$C_9$, $C_9$---$C_{10}$, $R^6$ and $R^8$ have the meanings given above, is reacted with an electrophilic reagent from the group chlorosulphonyl isocyanate, alkylthiosulphonium fluoroborate or acyl chloride, in the presence of a Lewis acid, and then, if desired, a carbonyl group in the 2-substituent is reduced with a complex metal hydride, or an alkylthio group is oxidised with periodate, or the acid addition salts are formed.

**5.** Process for the preparation of compounds of formula IB and their salts

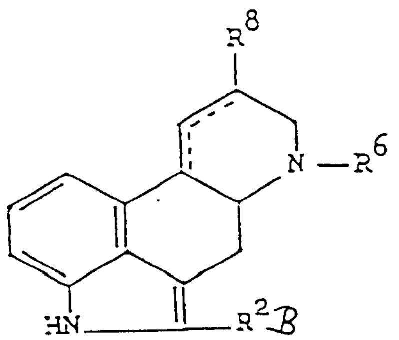

(IB)

in which
$C_8$---$C_9$ and $C_9$---$C_{10}$ represent a single bond or a double bond, but not a cumulative double bond, and the substituent in the 8-position is in the $\alpha$- or $\beta$-configuration when $C_8$---$C_9$ represents a single bond, and $C_8$---$C_9$ is a double bond when $R^8$ represents $CH_3$,
$R^6$ represents $C_{1-4}$-alkyl,
$R^8$ represents methyl, NH-CO-$NEt_2$ or NH-CS-$NEt_2$, and
$R^{2B}$ represents $C_{1-3}$-alkyl, hydroxyethyl, $C_{2-3}$-alkenyl, -COOH, -CONHR'', -CSNHR'', -COOR'', -S-$C_{1-4}$-alkyl or -$SiMe_2C_{1-4}$-alkyl, and R'' is $C_{1-4}$-alkyl,
characterised in that a compound of formula III

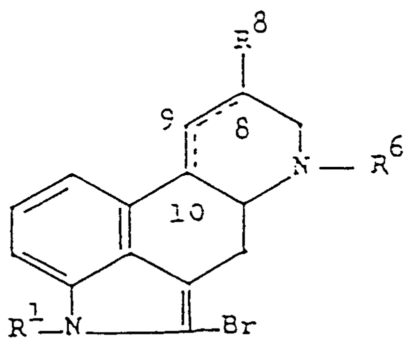

(III),

in which $C_8$---$C_9$, $C_9$---$C_{10}$, $R^6$ and $R^8$ have the meanings given above and $R^1$ represents a protecting group, is reacted with an alkyllithium or with phenyllithium at temperatures below 0 °C, and the resulting 2-Li-ergoline derivative of the general formula IV

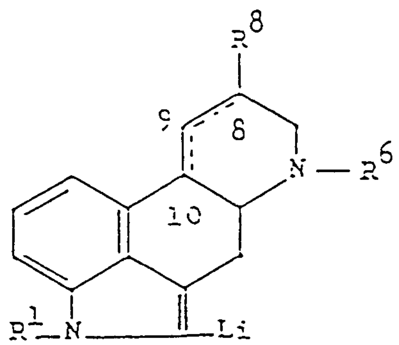

(IV),

in which $C_8$---$C_9$, $C_9$---$C_{10}$, $R^1$, $R^6$ and $R^8$ have the meanings given above, is reacted with an electrophilic reagent from the group carbon dioxide, ethylene oxide, $C_{1-4}$-alkylisocyanate, $C_{1-4}$-alkylthioisocyanate, $C_{1-4}$-alkyldimethylchlorosilane, $C_{1-3}$-alkyl and $C_{2-3}$-alkenyl bromide or iodide, or $C_{1-4}$-alkyldisulphides, at low temperatures of from -110 to -50 °C, in an inert solvent, and, if desired, a free 2-carboxy function is esterified by an aliphatic alcohol of the formula R"OH, or the protecting group $R^1$ is removed, or the acid addition salts are formed.

**6.** Process for the preparation of compounds of formula IC and their salts

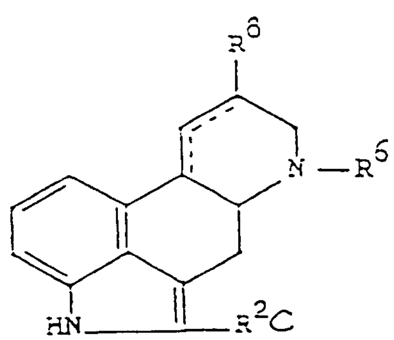

(IC)

in which

$C_8$---$C_9$ and $C_9$---$C_{10}$ represent a single bond or a double bond, but not a cumulative double bond, and the substituent in the 8-position is in the α- or β-configuration when $C_8$---$C_9$ represents a single bond, and $C_8$---$C_9$ is a double bond when $R^8$ represents $CH_3$,

$R^6$ represents $C_{1-4}$-alkyl,

$R^8$ represents methyl, NH-CO-$NEt_2$ or NH-CS-$NEt_2$, and

$R^{2C}$ represents the group COOH, CO-O-benzyl, CO-$NH_2$ or $CH_2$-$CH_2$-$CO_2$R" (R" = $C_{1-4}$-alkyl),

the group C≡C-R''' or HC = CH-R''' (R''' = hydrogen, $C_{1-4}$-alkyl, phenyl, $CH_2OH$, $CR''_2OH$,

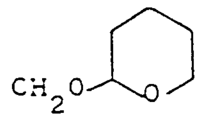 ,

$CO_2R''$, $CH_2NR''_2$, $SiMe_2R''$),
$C_{2-3}$-alkyl, phenyl-substituted ethyl or OH-substituted propyl,
characterised in that a compound of formula V

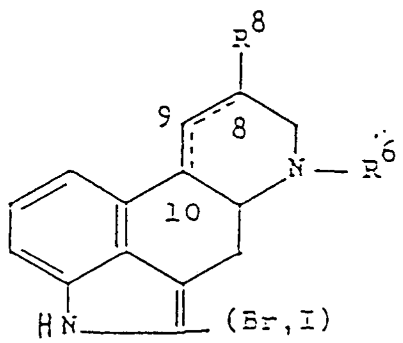

(V),

in which $C_8$---$C_9$, $C_9$---$C_{10}$, $R^6$ and $R^8$ have the meanings given above, is reacted with an electrophilic reagent from the group benzyl alcohol/carbon monoxide, acrylic ester $CH_2 = CH\text{-}CO_2R''$ or ethynyl derivative CH≡C-R''', wherein R'' and R''' have the meanings given above, in the presence of a palladium catalyst and of a secondary or tertiary amine, without a solvent or in an aprotic, water-miscible solvent, at temperatures above room temperature in the range of from 40 °C to the boiling temperature of the reaction mixture, and then, if desired, the 2-benzyl group is hydrogenated with palladium to form the carboxy compound or is reacted with ammonia to form the 2-carboxylic acid amide group, or an exocyclic multiple bond is hydrogenated with Raney nickel or palladium on a carrier to form the CC single bond or double bond, or an $SiMe_2R''$ protecting group is removed with a base or a tetrahydropyranyl protecting group is removed with pyridinium p-toluenesulphonate, or the acid addition salts are formed.

**7.** Process for the preparation of compounds of formula ID and their salts

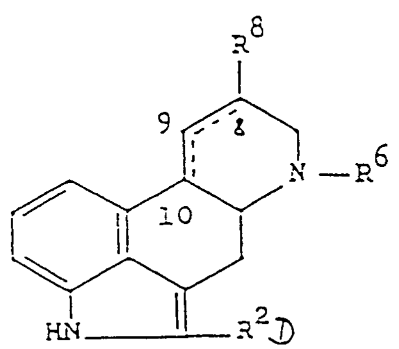

(ID)

in which
$C_8$---$C_9$ and $C_9$---$C_{10}$ represent a single bond or a double bond, but not a cumulative double bond, and the substituent in the 8-position is in the α- or β-configuration when $C_8$---$C_9$ represents a single bond, and $C_8$---$C_9$ is a double bond when $R^8$ represents $CH_3$,
$R^6$ represents $C_{1-4}$-alkyl,
$R^8$ represents methyl, $NH\text{-}CO\text{-}NEt_2$ or $NH\text{-}CS\text{-}NEt_2$, and
$R^{2D}$ is $SiMe_2$-$C_{1-4}$-alkyl,

characterised in that a compound of formula VI

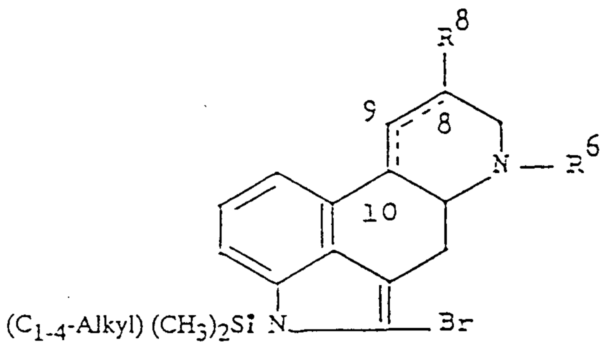

(VI),

in which $C_8$---$C_9$, $C_9$---$C_{10}$, $R^6$ and $R^8$ have the meanings given above, is reacted with an alkyllithium at temperatures below 0 °C, optionally in the presence of a tertiary amine, and, if desired, the acid addition salts are formed.

**8.** Use of the compounds according to claims 1 to 3 for the preparation of a medicament.

**9.** Process for the preparation of a medicament, characterised in that one of the compounds according to claims 1 to 3 is mixed with customary carriers and adjuvants.

**Claims for the following Contracting State : AT**

**1.** Process for the preparation of compounds of formula IA and their salts

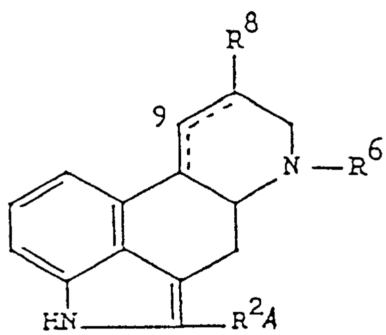

(IA)

in which
$C_8$---$C_9$ and $C_9$---$C_{10}$ represent a single bond or a double bond, but not a cumulative double bond, and the substituent in the 8-position is in the $\alpha$- or $\beta$-configuration when $C_8$---$C_9$ represents a single bond, and $C_8$---$C_9$ is a double bond when $R^8$ represents $CH_3$,
$R^6$ represents $C_{1-4}$-alkyl,
$R^8$ represents methyl, $NH$-$CO$-$NEt_2$ or $NH$-$CS$-$NEt_2$, and
$R^{2A}$ is CN, SR, SOR, -CO-R or -CHOH-R, wherein R represents H or $C_{1-4}$-alkyl,
characterised in that a compound of formula II

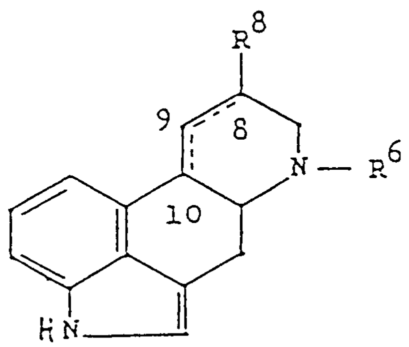

(II),

in which $C_8$---$C_9$, $C_9$---$C_{10}$, $R^6$ and $R^8$ have the meanings given above, is reacted with an electrophilic reagent from the group chlorosulphonyl isocyanate, alkylthiosulphonium fluoroborate or acyl chloride, in the presence of a Lewis acid, and then, if desired, a carbonyl group in the 2-substituent is reduced with a complex metal hydride, or an alkylthio group is oxidised with periodate, or the acid addition salts are formed.

**2.** Process for the preparation of compounds of formula IB and their salts

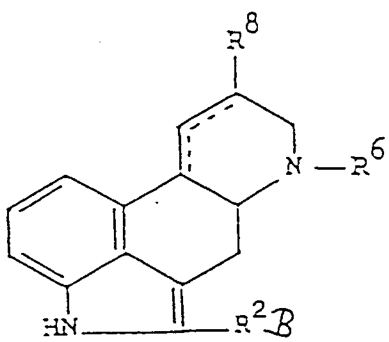

(IB)

in which
$C_8$---$C_9$ and $C_9$---$C_{10}$ represent a single bond or a double bond, but not a cumulative double bond, and the substituent in the 8-position is in the $\alpha$- or $\beta$-configuration when $C_8$---$C_9$ represents a single bond, and $C_8$---$C_9$ is a double bond when $R^8$ represents $CH_3$,
$R^6$ represents $C_{1-4}$-alkyl,
$R^8$ represents methyl, NH-CO-$NEt_2$ or NH-CS-$NEt_2$, and
$R^{2B}$ represents $C_{1-3}$-alkyl, hydroxyethyl, $C_{2-3}$-alkenyl, -COOH, -CONHR'', -CSNHR'', -COOR'', -S-$C_{1-4}$-alkyl or -$SiMe_2C_{1-4}$-alkyl, and R'' is $C_{1-4}$-alkyl,
characterised in that a compound of formula III

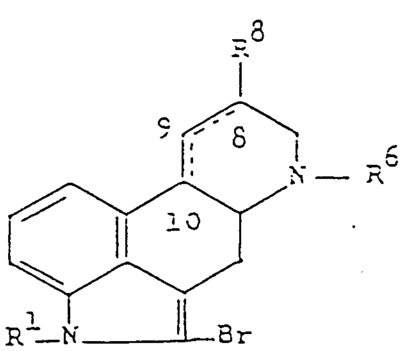

(III),

in which $C_8$---$C_9$, $C_9$---$C_{10}$, $R^6$ and $R^8$ have the meanings given above and $R^1$ represents a protecting

group, is reacted with an alkyllithium or with phenyllithium at temperatures below 0 °C, and the resulting 2-Li-ergoline derivative of the general formula IV

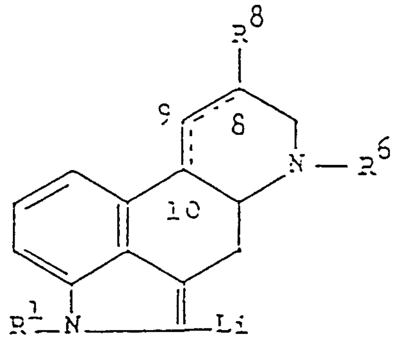

(IV),

in which $C_8$---$C_9$, $C_9$---$C_{10}$, $R^1$, $R^6$ and $R^8$ have the meanings given above, is reacted with an electrophilic reagent from the group carbon dioxide, ethylene oxide, $C_{1-4}$-alkylisocyanate, $C_{1-4}$-alkylthioisocyanate, $C_{1-4}$-alkyldimethylchlorosilane, $C_{1-3}$-alkyl and $C_{2-3}$-alkenyl bromide or iodide, or $C_{1-4}$-alkyldisulphides, at low temperatures of from -110 to -50 °C, in an inert solvent, and, if desired, a free 2-carboxy function is esterified by an aliphatic alcohol of the formula R"OH, or the protecting group $R^1$ is removed, or the acid addition salts are formed.

**3.** Process for the preparation of compounds of formula IC and their salts

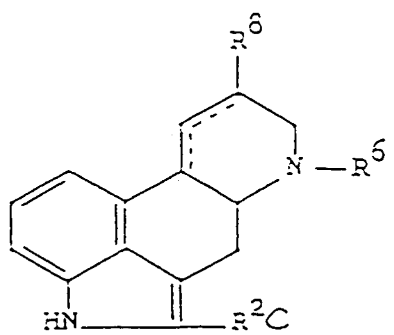

(IC)

in which
$C_8$---$C_9$ and $C_9$---$C_{10}$ represent a single bond or a double bond, but not a cumulative double bond, and the substituent in the 8-position is in the $\alpha$- or $\beta$-configuration when $C_8$---$C_9$ represents a single bond, and $C_8$---$C_9$ is a double bond when $R^8$ represents $CH_3$,
$R^6$ represents $C_{1-4}$-alkyl,
$R^8$ represents methyl, NH-CO-$NEt_2$ or NH-CS-$NEt_2$, and
$R^{2C}$ represents the group COOH, CO-O-benzyl, CO-$NH_2$ or $CH_2$-$CH_2$-$CO_2$R" (R" = $C_{1-4}$-alkyl),
the group C≡C-R"' or HC=CH-R"' (R"' = hydrogen, $C_{1-4}$-alkyl, phenyl, $CH_2OH$, $CR''_2OH$,

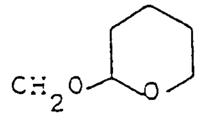

,

$CO_2R''$, $CH_2NR''_2$, $SiMe_2R''$),
$C_{2-3}$-alkyl, phenyl-substituted ethyl or OH-substituted propyl,
characterised in that a compound of formula V

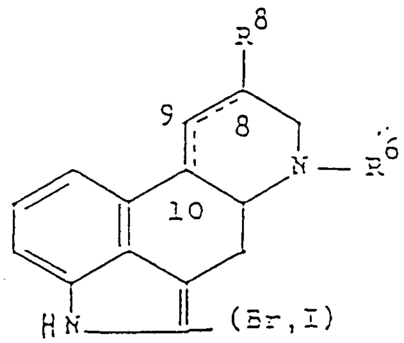

(V),

in which $C_8$---$C_9$, $C_9$---$C_{10}$, $R^6$ and $R^8$ have the meanings given above, is reacted with an electrophilic reagent from the group benzyl alcohol/carbon monoxide, acrylic ester $CH_2=CH\text{-}CO_2R''$ or ethynyl derivative $CH{\equiv}C\text{-}R'''$, wherein R'' and R''' have the meanings given above, in the presence of a palladium catalyst and of a secondary or tertiary amine, without a solvent or in an aprotic, water-miscible solvent, at temperatures above room temperature in the range of from 40 °C to the boiling temperature of the reaction mixture, and then, if desired, the 2-benzyl group is hydrogenated with palladium to form the carboxy compound or is reacted with ammonia to form the 2-carboxylic acid amide group, or an exocyclic multiple bond is hydrogenated with Raney nickel or palladium on a carrier to form the CC single bond or double bond, or an $SiMe_2R''$ protecting group is removed with a base or a tetrahydropyranyl protecting group is removed with pyridinium p-toluenesulphonate, or the acid addition salts are formed.

**4.** Process for the preparation of compounds of formula ID and their salts

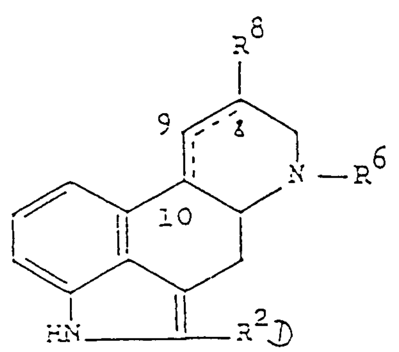

(ID)

in which

$C_8$---$C_9$ and $C_9$---$C_{10}$ represent a single bond or a double bond, but not a cumulative double bond, and the substituent in the 8-position is in the $\alpha$- or $\beta$-configuration when $C_8$---$C_9$ represents a single bond, and $C_8$---$C_9$ is a double bond when $R^8$ represents $CH_3$,

$R^6$ represents $C_{1-4}$-alkyl,

$R^8$ represents methyl, NH-CO-$NEt_2$ or NH-CS-$NEt_2$, and

$R^{2D}$ is $SiMe_2$-$C_{1-4}$-alkyl,

characterised in that a compound of formula VI

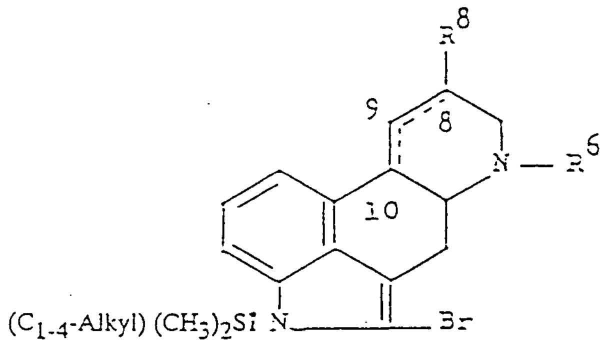

(VI),

in which $C_8$---$C_9$, $C_9$---$C_{10}$, $R^6$ and $R^8$ have the meanings given above, is reacted with an alkyllithium at temperatures below 0 °C, optionally in the presence of a tertiary amine, and, if desired, the acid addition salts are formed.

**5.** Process for the preparation of compounds of formula I and their salts

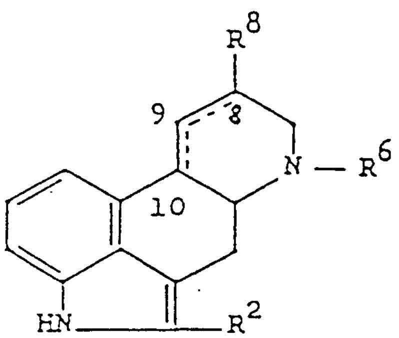

(I)

in which
$C_8$---$C_9$ and $C_9$---$C_{10}$ represent a CC single bond or a C=C double bond, but not a cumulative double bond, and the substituent in the 8-position is in the $\alpha$- or $\beta$-configuration when $C_8$---$C_9$ represents a CC single bond,
$R^2$ represents CN, SR, SOR,

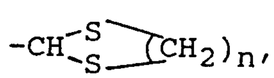

wherein n = 2 or 3,

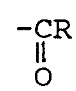

or -C(HOH)R, wherein R represents H or $C_1$-$C_4$-alkyl,
the group COR' or CSR' (R' = OH, $OC_{1-4}$-alkyl, benzyloxy, $NH_2$ or NHR''),
the group $CH_2$-$CH_2$-$CO_2$R'' (R'' = $C_{1-4}$-alkyl),
the group C≡C-R''' or HC=CH-R''' (R''' = hydrogen, $C_{1-4}$-alkyl, phenyl, $CH_2OH$, $CR''_2OH$,

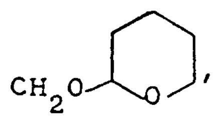

$CO_2R''$, $CH_2NR''_2$, $SiMe_2R''$),
$C_{1-3}$-alkyl that may be substituted by OH or by phenyl, or

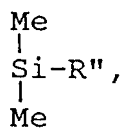

wherein R'' represents $C_{1-4}$-alkyl,
$R^6$ represents $C_{1-4}$-alkyl, and
$R^8$ represents methyl or the group $NH-CO-NEt_2$ or $NH-CS-NEt_2$,
and their salts, $C_8$---$C_9$ representing a double bond when $R^8$ is $CH_3$.
procuced by analogy with the procedures according to claims I - 4.

**6.** Process for the preparation of a medicament, characterised in that one of the compounds according to claims 1 to 5 is mixed with customary carriers and adjuvants.

## Revendications

**Revendications pour les Etats contractants suivants : BE, CH, DE, FR, GB, IT, LI, LU, NL, SE**

**1.** Dérivés d'ergolines substitués à la position 2, de formule générale I ci-dessous :

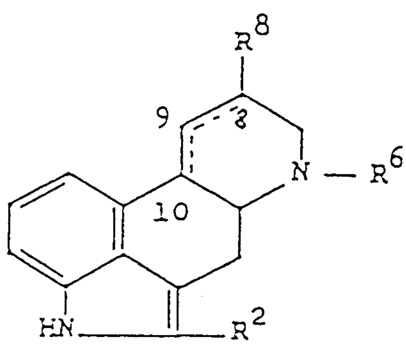

(I),

dans laquelle

| | |
|---|---|
| $C_8$---$C_9$ et $C_9$---$C_{10}$ | représentent une liaison simple CC ou une liaison double C=C mais pas une liaison double les deux à la fois et le substituant à la position 8 est en orientation $\alpha$ ou $\beta$ si $C_8$---$C_9$ est une liaison simple CC, |
| $R^2$ | représente un groupe CN, SR, SOR, |

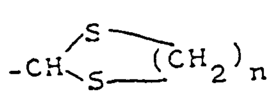

n étant le nombre 2 ou 3,

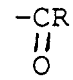

ou -C(HOH)R, R étant l'hydrogène ou un alkyle en $C_{1-4}$,
un groupe COR' ou CSR'
(R' étant OH, $OC_{1-4}$-alkyle, benzyloxy, $NH^2$ ou NHR"), un groupe $CH_2$-$CH_2$-$CO_2R$" (R" étant un alkyle en $C_{1-4}$),
un groupe C≡CR"' ou HC=CH-R"' (R"' étant hydrogène, $C_{1-4}$-alkyle, phényle, $CH_2OH$, $CR"_2$-OH,

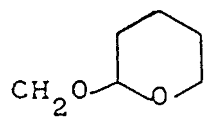,

$CO_2R$", $CH_2NR"_2$, $SiMe_2R$")
un alkyle en $C_{1-3}$ pouvant porter un groupe OH ou phényle,

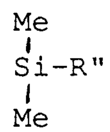,

R" étant un alkyle en $C_{1-4}$,

$R^6$ représente un alkyle en $C_{1-4}$, et

$R^8$ représente un méthyle ou le groupe NH-CO-$NEt_2$ ou NH-CS-$NEt_2$,

ainsi que leurs sels, $C_8$---$C_9$ étant une liaison double si $R^8$ est le groupe méthyle.

**2.** 3-(9,10-Didéshydro-6-méthyl-2-méthylthio-8α-ergolinyl)-1.1-diéthylurée et son tartrate.
3-(6-Méthyl-2-méthylthio-8α-ergolinyl)-1.1-diéthylurée et son tartrate.
1.1-Diéthyl-(6-méthyl-2-propyl-8α-ergolinyl)-urée
1.1-Diéthyl-(6-méthyl-2-isopropyl-8α-ergolinyl)-urée
8α-(3.3-Diéthyluréido)-2.6-diméthyl-ergoline
1.1-Diéthyl-(6-méthyl-2-éthynyl-8α-ergolinyl)-urée
1.1-Diéthyl-(6-méthyl-2-éthylthio-8α-ergolinyl)-urée
selon la revendication 1

**3.** 3-(2-Ethynyl-11.6-diméthyl-8α-ergolinyl)-1.1-diéthylurée.

**4.** Procédé de préparation de composés de formule IA ci-dessous et de leurs sels

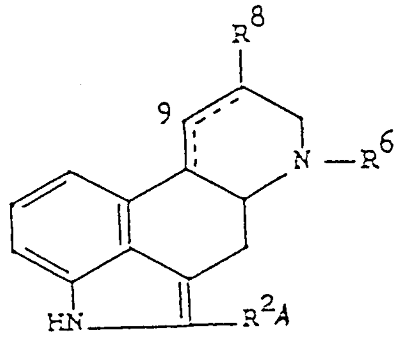

IA

dans laquelle

| | |
|---|---|
| $C_8$---$C_9$ et $C_9$---$C_{10}$ | représentent une liaison simple ou une une liaison double mais pas une liaison double les deux à la fois et le substituant à la position 8 est en orientation $\alpha$ ou $\beta$ si $C_8$---$C_9$ est une liaison simple, et $C_8$---$C_9$ une liaison double si $R^8$ est le groupe méthyle, |
| $R^6$ | représente un alkyle en $C_{1-4}$, |
| $R^8$ | un méthyle ou le groupe NH-CO-NEt$_2$ ou NH-CS-NEt$_2$, et |
| $R^{2A}$ | un groupe CN, SR, SOR, -CO-R ou -CHOH-R, R étant l'hydrogène ou un alkyle en $C_{1-4}$, |

procédé caractérisé en ce que l'on fait réagir un composé de formule II :

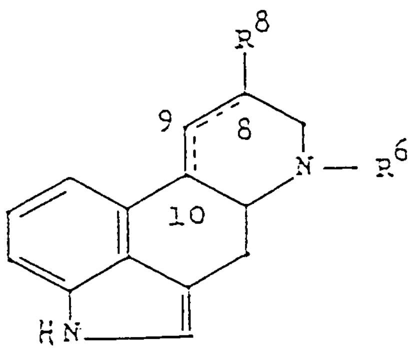

(II),

les divers symboles ayant les significations précédentes, avec un réactif électrophile pris parmi un isocyanate de chlorosulfonyle, un fluoroborate d'alkylthio-sulfonium et un chlorure d'acyle, en présence d'un acide de Lewis, puis éventuellement on réduit un groupe carbonyle du substituant 2 avec un hydrure de métal complexe ou on oxyde un groupe alkylthio avec un periodate ou encore ou forme des sels d'addition d'acides.

**5.** Procédé de préparation de composés de formule IB et de leurs sels :

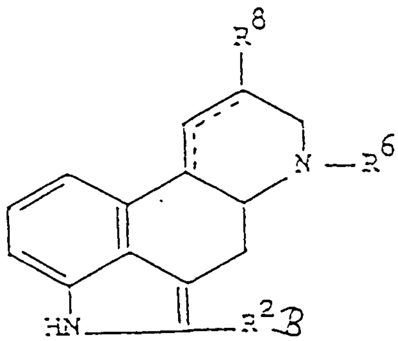

IB

dans laquelle

| | |
|---|---|
| $C_8$---$C_9$ et $C_9$---$C_{10}$ | représentent une liaison simple ou une double mais pas une liaison double les deux à la fois et le substituant à la position 8 est en orientation $\alpha$ ou $\beta$ si $C_8$---$C_9$ est une liaison simple et $C_8$---$C_9$ est une liaison double si $R^8$ est le groupe méthyle, |
| $R^6$ | représente un alkyle en $C_{1-4}$, |
| $R^8$ | un méthyle ou le groupe NH-CO-NEt$_2$ ou NH-CS-NEt$_2$, et |
| $R^{2B}$ | un alkyle en $C_{1-3}$, hydroxyéthyle, alcényle en $C_{2-3}$, -COOH, -CONHR'', -CSNHR'', -S-$C_{1-4}$-alkyle ou -SiMe$C_{1-4}$-alkyle, R'' étant un alkyle en $C_{1-4}$, |

procédé caractérisé en ce que l'on fait réagir un composé de formule III :

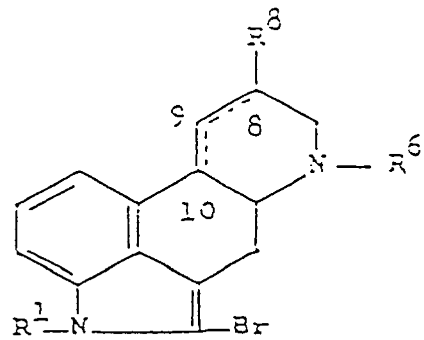

(III),

les divers symboles ayant les significations précédentes et R1 étant un groupe protecteur, avec un lithium-alkyle ou le lithium-phényle à une température inférieure à 0 ° C, puis on fait réagir la 2-Li-ergoline, ainsi obtenue, de formule générale IV :

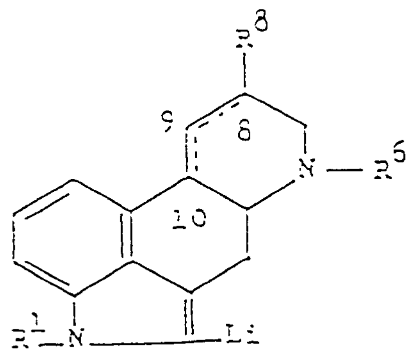

(IV),

avec un réactif électrophile pris parmi le dioxyde de carbone, l'oxyde d'éthylène, un isocyanate ou un thioisocyanate d'alkyle en $C_{1-4}$, un $C_{1-4}$-alkyldiméthylchlorosilane,un bromure ou iodure d'alkyle en $C_{1-3}$ ou d'alcényle en $C_{2-3}$ et un disulfure d'alkyle en $C_{1-4}$, à des températures de -110 à -50 ° C dans un solvant inerte, et le cas échéant on estérifie un groupe 2-carboxylique libre avec un alcool aliphatique R''OH ou on élimine le groupe protecteur $R^1$ ou encore on forme les sels d'addition d'acides.

**6.** Procédé de préparation de composés de formule IC et de leurs sels :

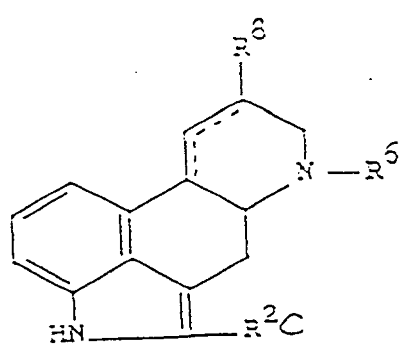

IC

formule dans laquelle

$C_8 \underline{---} C_9$ et $C_9 \underline{---} C_{10}$ représentent une liaison simple ou double mais pas une double liaison les deux à la fois et le substituant en position 8 est en orientation $\alpha$ ou $\beta$ si $C_8 \underline{---} C_9$ est une liaison simple et $C_8 \underline{---} C_9$ est une double liaison si $R^8$ est le

groupe méthyle,

$R^6$ représente un alkyle en $C_{1-4}$,

$R^8$ le groupe méthyle, $NH-CO-NEt_2$ ou $NH-CS-NEt_2$, et

$R^{2C}$ un groupe -COOH, CO-O-benzyle, CO-NH2 ou $CH_2CH_2-CO_2R''$ (R'' étant un alkyle en $C_{1-4}$), un groupe $C{\equiv}C-R'''$ ou $HC=CH-R'''$ (R''' étant l'hydrogène, un alkyle en $C_{1-4}$, un phényle, $CH_2OH$, $CR_2''OH$,

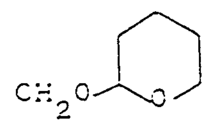 ,

$CO_2R''$, $CH_2NR''_2$, $SiMe_2R''$),
un alkyle en $C_{2-3}$, un phényléthyle ou un hydroxypropyle,

procédé caractérisé en ce qu'on fait réagir un composé de formule V :

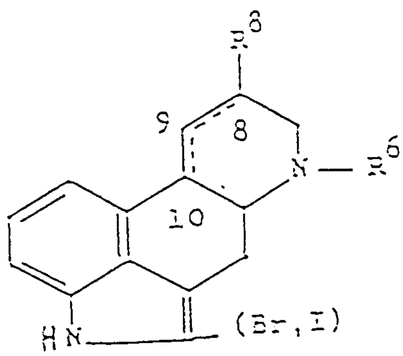

(Y),

les divers symboles ayant les significations précédentes, avec un réactif électrophile pris parmi l'alcool benzylique avec le monoxyde de carbone, un ester acrylique $CH_2=CH-CO_2R''$ et un dérivé éthynylique $CH{\equiv}CR'''$ dont R'' et R''' ont les significations précédentes, en présence d'un catalyseur de palladium et d'une amine tertiaire ou secondaire, sans solvant ou dans un solvant aprotique, avec un solvant miscible à l'eau, à des températures supérieures à la température ambiante comprises entre 40 °C et la température d'ébullition du mélange de réaction, puis le cas échéant on hydrogène le groupe 2-benzylique en présence de palladium avec formation du composé carboxylique ou on forme le groupe 2-carboxylamide avec de l'ammoniac ou encore on hydrogène une liaison multiple exocyclique en une liaison simple ou double avec du nickel Raney ou du palladium sur support, ou on élimine un groupe protecteur $SiMe_2R''$ avec une base ou un groupe protecteur tétrahydropyrannyle avec du p-toluène-sulfonate de pyridinium, ou bien on forme les sels d'addition d'acides.

**7.** Procédé de préparation de composés de formule ID et de leurs sels :

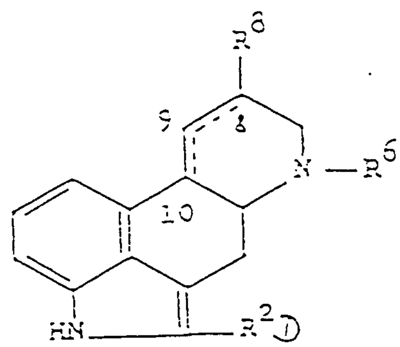

ID.

formule dans laquelle

$C_8$---$C_9$ et $C_9$---$C_{10}$ représentent une liaison simple ou double mais pas une double liaison les deux à la fois et le substituant en position 8 a l'orientation $\alpha$ ou $\beta$ si $C_8$---$C_9$ est une liaison simple et $C_8$---$C_9$ est une double liaison si $R^8$ est le groupe méthyle,

$R^6$ est un alkyle en $C_{1-4}$,

$R^8$ le groupe méthyle, NH-CO-$NEt_2$ ou NH-CS-$NEt_2$, et

$R^{2D}$ un $SiMe_2R''$-$C_{1-4}$-alkyle,

procédé caractérisé en ce qu'on fait réagir un composé de formule VI :

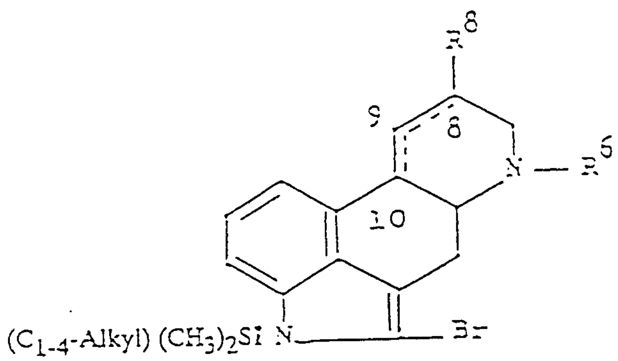

(VI),

les divers symboles ayant les significations précédentes, avec un lithium-alkyle à des températures inférieures à 0 °C, éventuellement en présence d'une amine tertiaire, et si l'on veut on forme les sels d'addition d'acides.

**8.** Emploi des composés des revendications 1 à 3 pour la fabrication de médicaments.

**9.** Procédé de fabrication de médicaments, procédé caractérisé en ce que l'on mélange l'un des composés des revendications 1 à 3 avec des véhicules et excipients courants.

**Revendications pour l'Etat contractant suivant : AT**

**1.** Procédé de préparation de composés de formule IA ci-dessous et de leurs sels

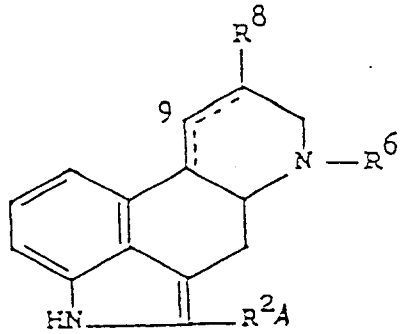

IA

dans laquelle

| | |
|---|---|
| $C_8$---$C_9$ et $C_9$---$C_{10}$ | représentent une liaison simple ou une une liaison double mais pas une liaison double les deux à la fois et le substituant à la position 8 est en orientation $\alpha$ ou $\beta$ si $C_8$---$C_9$ est une liaison simple, et $C_8$---$C_9$ une liaison double si $R^8$ est le groupe méthyle, |
| $R^6$ | représente un alkyle en $C_{1-4}$, |
| $R^8$ | un méthyle ou le groupe NH-CO-$NEt_2$ ou NH-CS-$NEt_2$, et |
| $R^{2A}$ | un groupe CN, SR, SOR, -CO-R ou -CHOH-R, R étant l'hydrogène ou un alkyle en $C_{1-4}$, |

procédé caractérisé en ce que l'on fait réagir un composé de formule II :

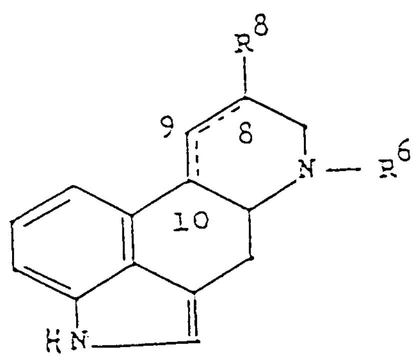

(II),

les divers symboles ayant les significations précédentes, avec un réactif électrophile pris parmi un isocyanate de chlorosulfonyle, un fluoroborate d'alkylthio-sulfonium et un chlorure d'acyle, en présence d'un acide de Lewis, puis éventuellement on réduit un groupe carbonyle du substituant 2 avec un hydrure de métal complexe ou on oxyde un groupe alkylthio avec un periodate ou encore ou forme des sels d'addition d'acides.

**2.** Procédé de préparation de composés de formule IB et de leurs sels :

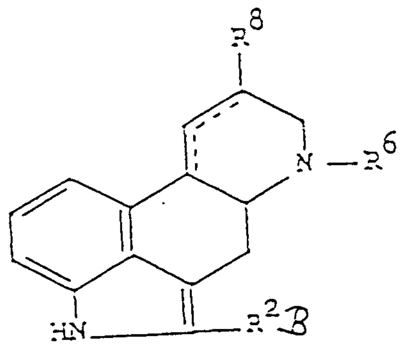

IB

dans laquelle

$C_8$---$C_9$ or $C_9$---$C_{10}$ représentent une liaison simple ou une double mais pas une liaison double les deux à la fois et le substituant à la position 8 est en orientation $\alpha$ ou $\beta$ si $C_8$---$C_9$ est une liaison simple et $C_8$---$C_9$ est une liaison double si $R^8$ est le groupe méthyle,

$R^6$ représente un alkyle en $C_{1\ 4}$,

$R^8$ un méthyle ou le groupe NH-CO-$NEt_2$ ou NH-CS-$NEt_2$, et

$R^{2B}$ un alkyle en $C_{1-3}$, hydroxyéthyle, alcényle en $C_{2-3}$, -COOH,-CONHR", -CSNHR", -S-$C_{1-4}$-alkyle ou -SiMe$C_{1-4}$-alkyle, R" étant un alkyle en $C_{1-4}$,

procédé caractérisé en ce que l'on fait réagir un composé de formule III :

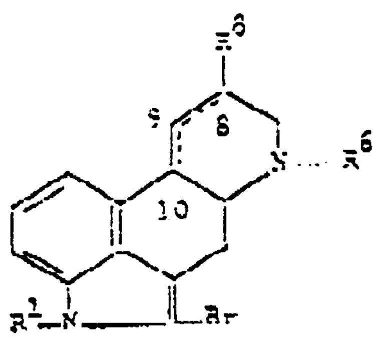

(III)

les divers symboles ayant les significations précédentes et R1 étant un groupe protecteur, avec un lithium-alkyle ou le lithium-phényle à une température inférieure à 0 °C, puis on fait réagir la 2-Li ergoline, ainsi obtenue, de formule générale IV :

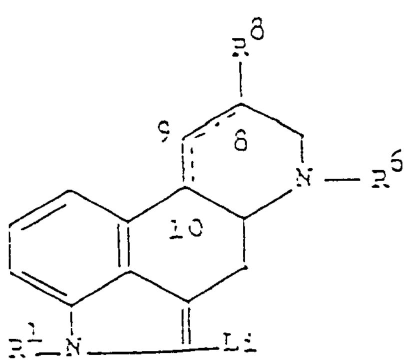

(IV),

avec un réactif électrophile pris parmi le dioxyde de carbone, l'oxyde d'éthylène, un isocyanate ou un thioisocyanate d'alkyle en $C_{1-4}$, un $C_{1-4}$-alkyldiméthylchlorosilane,un bromure ou iodure d'alkyle en

$C_{1-3}$ ou d'alcényle en $C_{2-3}$ et un disulfure d'alkyle en $C_{1-4}$, à des températures de -110 à -50 °C dans un solvant inerte, et le cas échéant on estérifie un groupe 2-carboxylique libre avec un alcool aliphatique R''OH ou on élimine le groupe protecteur $R^1$ ou encore on forme les sels d'addition d'acides.

**3.** Procédé de préparation de composés de formule IC et de leurs sels :

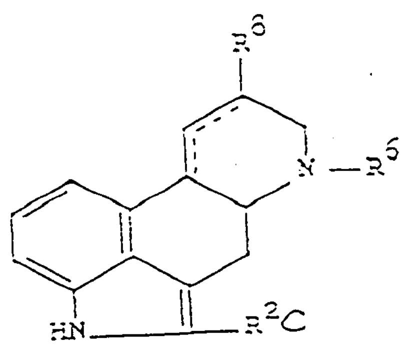

IC

formule dans laquelle

- $C_8$---$C_9$ et $C_9$---$C_{10}$ : représentent une liaison simple ou double mais pas une double liaison les deux à la fois et le substituant en position 8 est en orientation $\alpha$ ou $\beta$ si $C_8$---$C_9$ est une liaison simple et $C_8$---$C_9$ est une double liaison si $R^8$ est le groupe méthyle,
- $R^6$ : représente un alkyle en $C_{1-4}$,
- $R^8$ : le groupe méthyle, $NH\text{-}CO\text{-}NEt_2$ ou $NH\text{-}CS\text{-}NEt_2$, et
- $R^{2C}$ : un groupe -COOH, CO-O-benzyle, CO-NH2 ou $CH_2CH_2\text{-}CO_2R''$ (R'' étant un alkyle en $C_{1-4}$), un groupe $C\equiv C\text{-}R'''$ ou $HC=CH\text{-}R'''$ (R''' étant l'hydrogène, un alkyle en $C_{1-4}$, un phényle, $CH_2OH$, $CR_2''OH$,

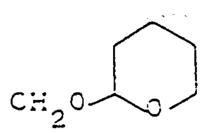

,

$CO_2R''$, $CH_2NR''_2$, $SiMe_2R''$), un alkyle en $C_{2-3}$, un phényléthyle ou un hydroxypropyle,

procédé caractérisé en ce qu'on fait réagir un composé de formule V :

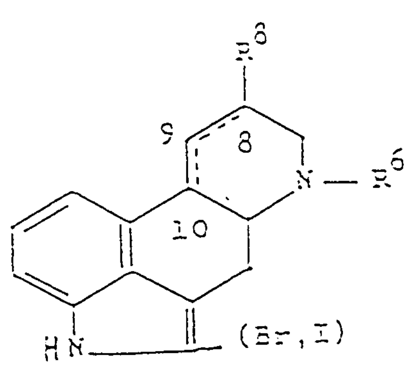

(V),

les divers symboles ayant les significations précédentes, avec un réactif électrophile pris parmi l'alcool benzylique avec le monoxyde de carbone, un ester acrylique $CH_2 = CH\text{-}CO_2R''$ et un dérivé éthynylique $CH{\equiv}CR'''$ dont R'' et R''' ont les significations précédentes, en présence d'un catalyseur de palladium et d'une amine tertiaire ou secondaire, sans solvant ou dans un solvant aprotique, avec un solvant miscible à l'eau, à des températures supérieures à la température ambiante comprises entre 40 °C et la température d'ébullition du mélange de réaction, puis le cas échéant on hydrogène le groupe 2-benzylique en présence de palladium avec formation du composé carboxylique ou on forme le groupe 2-carboxylamide avec de l'ammoniac ou encore on hydrogène une liaison multiple exocyclique en une liaison simple ou double avec du nickel Raney ou du palladium sur support, ou on élimine un groupe protecteur $SiMe_2R''$ avec une base ou un groupe protecteur tétrahydropyrannyle avec du p-toluènesulfonate de pyridinium, ou bien on forme les sels d'addition d'acides.

**4.** Procédé de préparation de composés de formule ID et de leurs sels :

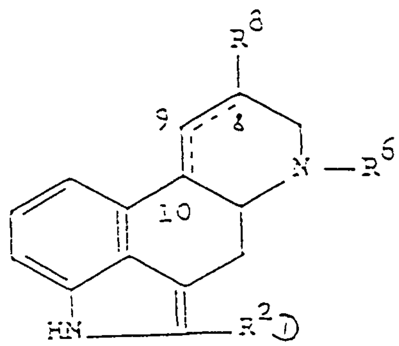

ID.

formule dans laquelle

| | |
|---|---|
| $C_8{-}{-}{-}C_9$ et $C_9{-}{-}{-}C_{10}$ | représentent une liaison simple ou double mais pas une double liaison les deux à la fois et le substituant en position 8 a l'orientation α ou β si $C_8{-}{-}{-}C_9$ est une liaison simple et $C_8{-}{-}{-}C_9$ est une double liaison si $R^8$ est le groupe méthyle, |
| $R^6$ | est un alkyle en $C_{1-4}$, |
| $R^8$ | le groupe méthyle, NH-CO-$NEt_2$ ou NH-CS-$NEt_2$, et |
| $R^{2D}$ | un $SiMe_2R''$-$C_{1-4}$-alkyle, |

procédé caractérisé en ce qu'on fait réagir un composé de formule VI :

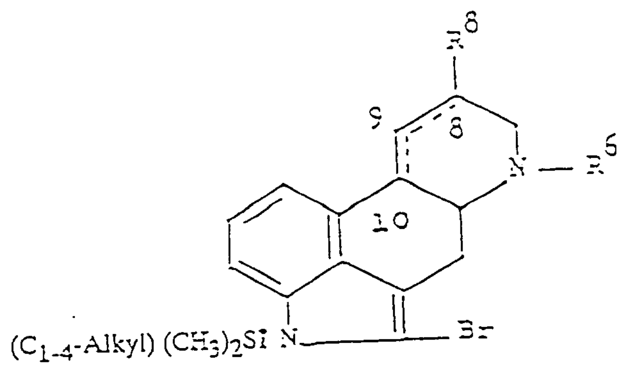

(VI),

les divers symboles ayant les significations précédentes, avec un lithium-alkyle à des températures inférieures à 0 °C, éventuellement en présence d'une amine tertiaire,
et si l'on veut on forme les sels d'addition d'acides.

**5.** Procédé de préparation de composés de formule I ci-dessous et de leurs sels

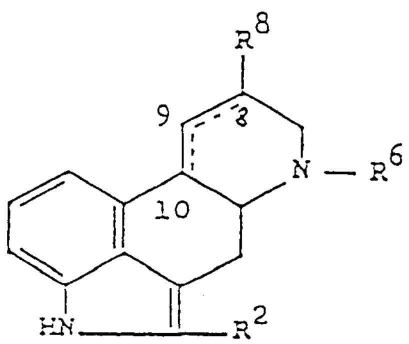

(I),

dans laquelle

$C_8$---$C_9$ et $C_9$---$C_{10}$ représentent une liaison simple CC ou une liaison double C = C mais pas une liaison double les deux à la fois et le substituant à la position 8 est en orientation $\alpha$ ou $\beta$ si $C_8$---$C_9$ est une liaison simple CC,

$R^2$ représente un groupe CN, SR, SOR,

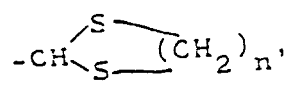

n étant le nombre 2 ou 3,

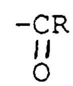

ou -C(HOH)R, R étant l'hydrogène ou un alkyle en $C_{1-4}$,
un groupe COR' ou CSR'
(R' étant OH, $OC_{1-4}$-alkyle, benzyloxy, $NH^2$ ou NHR''),
un groupe $CH_2$-$CH_2$-$CO_2R''$ (R'' étant un alkyle en $C_{1-4}$),
un groupe C≡CR''' ou HC = CH-R''' (R''' étant hydrogène, $C_{1-4}$-alkyle, phényle, $CH_2OH$, $CR''_2$-OH,

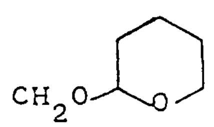

,

$CO_2R''$, $CH_2NR''_2$, $SiMe_2R''$)
un alkyle en $C_{1-3}$ pouvant porter un groupe OH ou phényle,

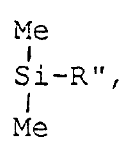

R'' étant un alkyle en $C_{1-4}$,

$R^6$ représente un alkyle en $C_{1-4}$, et

$R^8$ représente un méthyle ou le groupe NH-CO-$NEt_2$ ou NH-CS-$NEt_2$,

ainsi que leurs sels, $C_8$---$C_9$ étant une liaison double si $R^8$ est le groupe méthyle, preparées analogue

les revendications I - 4.

**6.** Procédé de fabrication de médicaments, procédé caractérisé en ce que l'on mélange l'un des composés des revendications 1 à 5 avec des véhicules et excipients courants.